(19) Europäisches Patentamt
European Patent Office
Office européen des brevets

(11) EP 4 450 095 A1

(12) **EUROPEAN PATENT APPLICATION**
published in accordance with Art. 153(4) EPC

(43) Date of publication:
**23.10.2024 Bulletin 2024/43**

(21) Application number: **22906081.9**

(22) Date of filing: **27.10.2022**

(51) International Patent Classification (IPC):
***A61L 24/04*** *(2006.01)* ***A61L 27/50*** *(2006.01)*
***A61L 27/26*** *(2006.01)* ***C08L 71/02*** *(2006.01)*
***A61L 24/00*** *(2006.01)* ***A61L 27/52*** *(2006.01)*
***C08J 3/075*** *(2006.01)*

(52) Cooperative Patent Classification (CPC):
**A61L 24/00; A61L 24/04; A61L 27/26; A61L 27/50; A61L 27/52; C08J 3/075; C08L 71/02;** Y02A 50/30

(86) International application number:
**PCT/CN2022/127941**

(87) International publication number:
**WO 2023/109329 (22.06.2023 Gazette 2023/25)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **13.12.2021 CN 202111520296**

(71) Applicant: **Beijing Biosis Healing Biological Technology Co., Ltd.**
**Beijing 102600 (CN)**

(72) Inventors:
- **ZHAO, Bo**
  **Beijing 102600 (CN)**
- **WEI, Pengfei**
  **Beijing 102600 (CN)**
- **HUANG, Yiqian**
  **Beijing 102600 (CN)**
- **JING, Wei**
  **Beijing 102600 (CN)**

(74) Representative: **Perani & Partners S.p.A.**
**Piazza Armando Diaz, 7**
**20123 Milano (IT)**

(54) **MEDICAL TISSUE ADHESIVE, AND PREPARATION METHOD THEREFOR AND USE THEREOF**

(57) The present disclosure belongs to the field of biomedical materials, and specifically relates to a medical tissue adhesive, and a preparation method therefor and the use thereof, and a composition and a kit for preparing the medical tissue adhesive. According to the medical tissue adhesive provided by the present disclosure, a three-dimensional net-shaped gel skeleton structure is formed by means of the mutual crosslinking of a polyethylene glycol derivative I, a polyethylene glycol derivative II and trilysine. Moreover, the gel skeleton has a crosslinked network density and intermolecular interaction that can be improved, and accordingly has an improved structure bonding strength and mechanical strength. In addition, the medical tissue adhesive has the advantages of a reduced swelling rate, a high biocompatibility and biodegradability, can effectively adhere to tissues and seal wounds, reduces compression on tissues such as nerves, realizes wound sealing, tissue repair and blocking of postoperative liquids such as blood and tissue fluid, and has great clinical significance and wide application prospects.

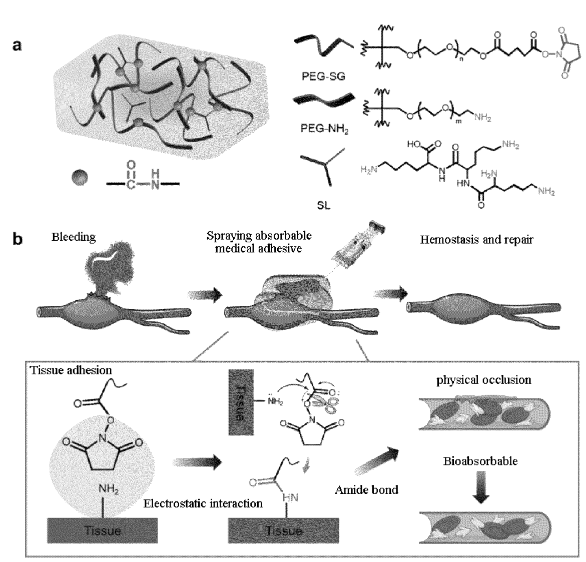


FIG. 1

EP 4 450 095 A1

## Description

### CROSS-REFERENCE TO RELATED APPLICATION

The present disclosure claims priority to Chinese Patent Application No. 202111520296.6 titled "MEDICAL TISSUE ADHESIVE, AND PREPARATION METHOD THEREFOR AND USE THEREOF" filed on December 13, 2021, the entire contents (including the appendixes) of which are incorporated herein by reference.

### TECHNICAL FIELD

The present disclosure belongs to the field of biomedical materials, and in particular, it relates to a medical tissue adhesive, a method of preparing the same, and use thereof, as well as to a composition and kit for preparing a medical tissue adhesive.

### BACKGROUND

Medical tissue adhesives are polymeric materials that are capable of adhesion with a certain degree of strength to skins or *in vivo* parts, and are chiefly used to bind interfaces between damaged or broken tissues for the purposes of hemostasis and inhibition of liquid or gas leakage. Medical tissue adhesives can adhere to biological tissues, and have been used more and more to replace or assist suturing or stapling to achieve wound closure purposes.

Medical tissue adhesives are mainly categorized as natural and synthetic. Natural tissue adhesives include fibrin-based ones and polysaccharide-based ones, among others; synthetic tissue adhesives include aldehyde-based complexes, cyanoacrylate, and polyethylene glycol modified products, among others. Natural tissue adhesives have played a significant role in many surgical operations, but they come from limited sources, have to undergo complicated preparation processes, and are plagued with some immunogenicity and anticoagulation. Synthetic tissue adhesives are favorable to hemostasis of anticoagulant patients, and also have the advantages of low costs, high reproducibility, easy manufacture, and customizability, so they are developing rapidly amid tissue adhesives.

Polyethylene glycol (PEG), as an important hydrophilic polymer with key properties such as satisfactory biocompatibility, non-immunogenicity, and tolerance to protein adsorption, has been widely used in the biomedicine field. Corporations at home and abroad have developed technical chains of PEG-related products based on the fact that PEG's structure allows for designing, and have made breakthroughs. For example, Coseal tissue adhesives, developed by Baxter using 4-arm PEG, was approved by the FDA for marketing in 2001. An absorbable *dura mater* sealant of a 4-arm PEG derivative that was launched by Shandong Saikesaisi Biotechnology Co., Ltd. in 2018 is a major one of PEG-modified tissue adhesives currently circulating in the clinical medicine market in China. The gelation and tissue adhesion mechanism of PEG-based tissue adhesives can be summarized as follows: The succinimidyl ester group in modified PEG molecule structure is so chemically active as to be capable of reacting with another substance and thereby forming a crosslinking network, which results in gelling. The succinimidyl ester group can also chemically interact with amino groups in a biological tissue and thereby achieve a tissue adhesion effect, but it has no adhesion to substances that are not biological tissues, such as metals, glass, and ceramics.

Although being favorable in biocompatibility and tissue adhesion, PEG tissue adhesives have some defects that limit their wide application. For example, PEG is a hydrophilic polymer, and a gel formed of it, when soaking in an aqueous solution or tissue fluid for a long time, usually swells to such a large extent that it may compress peripheral blood vessels and nerves and bring potential safety hazards. As another example, compared with natural polymers that have conjugated structures in their molecules, such as chitosan and gelatin, PEG is more flexible, so a gel formed of it has a lower modulus of elasticity than that of chitosan and gelatin. Thus, when it is used in emergent clinical scenarios such as hemorrhage of coronary arteries, the gel can be easily washed away by the high pressure from the liquid burst. That explains why, at present, it can only be used for auxiliary hemostasis and sealing after suturing.

In view of the above, it is urgent to develop PEG-modified tissue adhesives that avoid or even eliminate the shortcomings described above and can provide feasible methods and strategies for plain sailing of surgical operations and for health and safety of patients.

### SUMMARY

<u>Technical problems</u>

In view of the problems in the prior art, e.g., the problems of PEG tissue adhesives, including high swelling rate, low mechanical strength, insufficient adhesion to tissues, and incapability to close wounds, the present disclosure provides a medical tissue adhesive that has high mechanical strength, satisfactory adhesion to tissues, and low swelling.

To this end, the present disclosure provides a medical tissue adhesive that has a gel framework formed by bonding a polyethylene glycol derivative I with a polyethylene glycol derivative II and a trilysine respectively. The medical tissue adhesive has satisfactory biocompatibility, improved mechanical properties, reduced swelling rate, and biodegradability. It keeps sticky and adheres to different types of tissues in wet conditions, and thereby meets different biomedical requirements such as wound closure, tissue repair, and liquid occlusion. Therefore, it is of clinical significance and carries broad use prospects.

Solutions to problems

A first aspect of the present disclosure provides a medical tissue adhesive, which has a gel framework formed by bonding a polyethylene glycol derivative I with a polyethylene glycol derivative II and a trilysine respectively;

wherein a structure of the polyethylene glycol derivative I is shown in Formula (I):

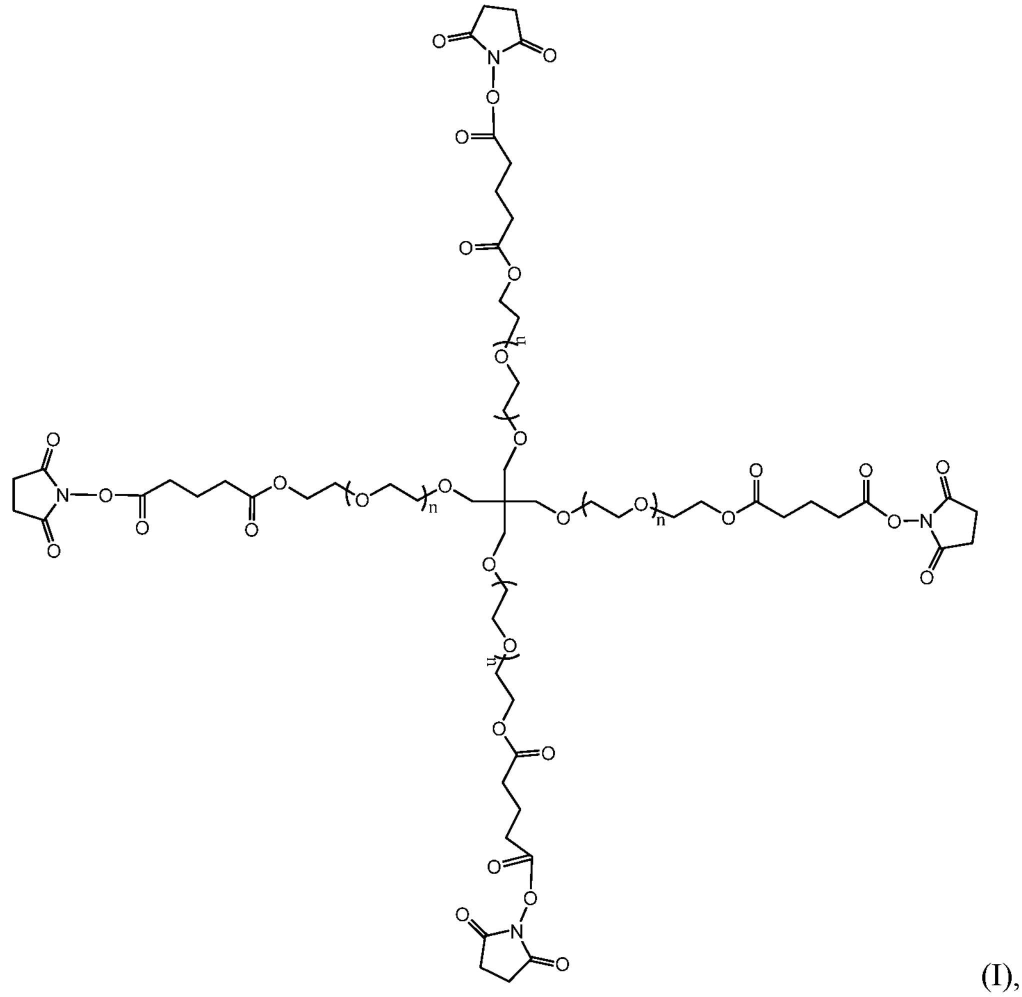

(I),

and

a structure of polyethylene glycol derivative II is shown in Formula (II):

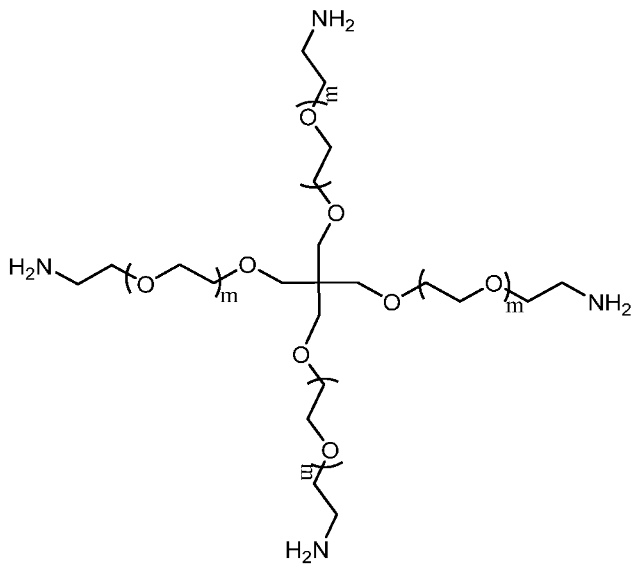

(II),

wherein m and n are each a natural number; a molecular weight of the polyethylene glycol derivative I is 2,000-20,000, preferably 5,000-15,000, and more preferably 8,000-12,000; a molecular weight of the polyethylene glycol derivative II is 2,000-20,000, preferably 5,000-15,000, and more preferably 8,000-12,000.

In some embodiments, in the medical tissue adhesive of the present disclosure, the mass ratio of the polyethylene glycol derivative I to the polyethylene glycol derivative II is 1: (0.8-1.2), preferably 1: (0.9-1.1), and more preferably 1: (0.95-1.05).

In some embodiments, in the medical hydrogel of the present disclosure, the mass ratio of the polyethylene glycol derivative I to trilysine is 1: (0.003-0.1), preferably 1: (0.005-0.05), and more preferably 1: (0.01-0.02).

A second aspect of the present disclosure provides a method of preparing the medical tissue adhesive according to the first aspect, which includes:

a step of preparing a solution A comprising a polyethylene glycol derivative I;
a step of preparing a solution B comprising a polyethylene glycol derivative II and trilysine; and
a step of mixing the solution A and the solution B, to obtain the medical tissue adhesive.

In some embodiments, in the method according to the present disclosure, the step of preparing a solution B comprising a polyethylene glycol derivative II and trilysine includes:

preparing a solution comprising the polyethylene glycol derivative II and a solution comprising trilysine respectively, and mixing the two solutions, to obtain the solution B; or
preparing a solution comprising the polyethylene glycol derivative II, and adding trilysine to the solution comprising the polyethylene glycol derivative II, to obtain the solution B; or,
preparing a solution comprising trilysine, and adding the polyethylene glycol derivative II to the solution comprising trilysine, to obtain the solution B; or
mixing the polyethylene glycol derivative II and trilysine, and adding them to a solvent, to obtain the solution B.

In some embodiments, in the method according to present disclosure, the solution B comprises the polyethylene glycol derivative II in a percent concentration by mass of 10%-30% (m/v), and preferably 18%-22% (m/v).

In some embodiments, in the method according to the present disclosure, the solution B comprises trilysine in a percent concentration by mass of 0.06%-2% (m/v), preferably 0.1%-1% (m/v), and more preferably 0.2%-0.4% (m/v).

In some embodiments, in the method according to the present disclosure, the solution A comprises the polyethylene glycol derivative I in a percent concentration by mass of 10%-30% (m/v), and preferably 18%-22% (m/v).

A third aspect of the present disclosure provides use of the medical tissue adhesive according to the first aspect or a medical tissue adhesive obtained by the method according to the second aspect as a biomedical material or in the preparation of a biomedical material; optionally, the biomedical material is at least one selected from (a)-(d):

(a) a medical adhesive for endoscope closure;

(b) a medical adhesive for blood vessel closure;
(c) a medical adhesive for surgery closure; and
(d) a medical adhesive for cerebrospinal fluid closure.

A fourth aspect of the present disclosure provides a composition for preparing a medical tissue adhesive, which includes:

a component E comprising a polyethylene glycol derivative I with a structure as shown in Formula (I):

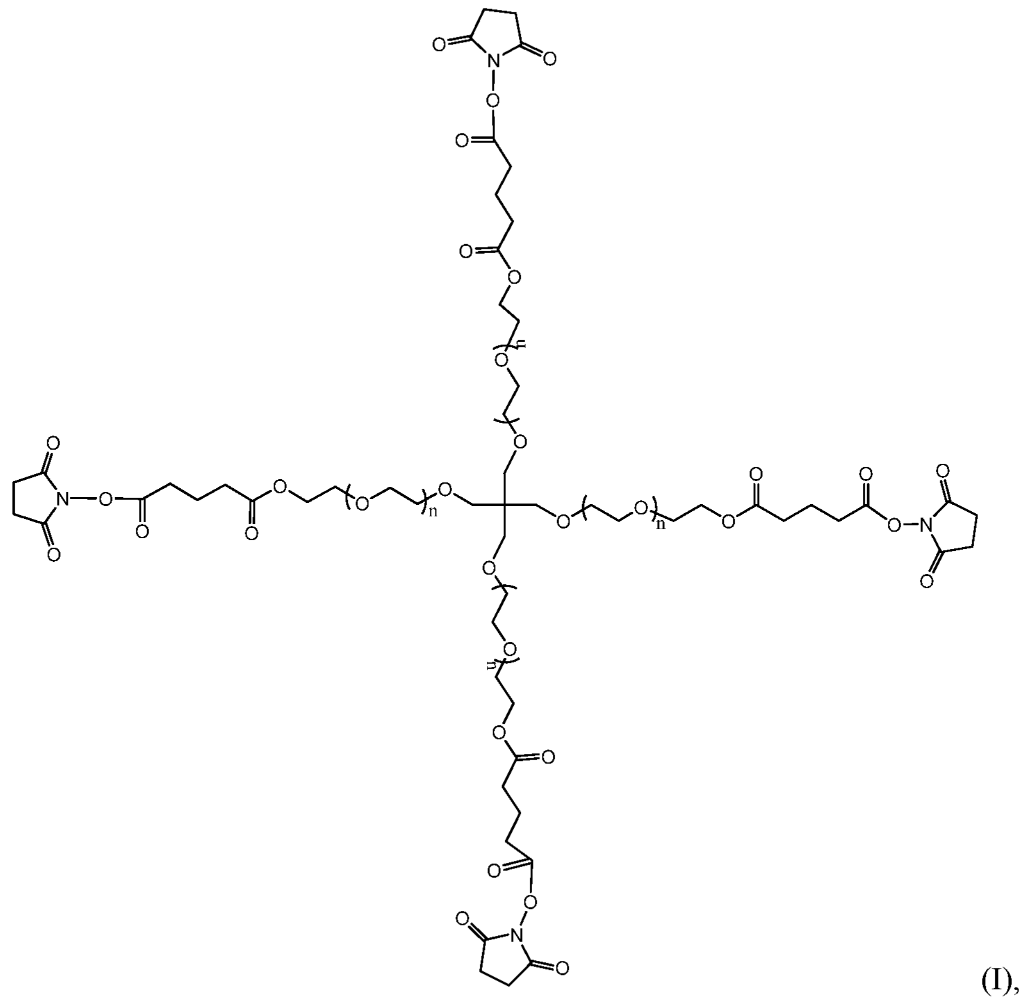

(I),

a component F comprising a polyethylene glycol derivative II with a structure as shown in Formula (II):

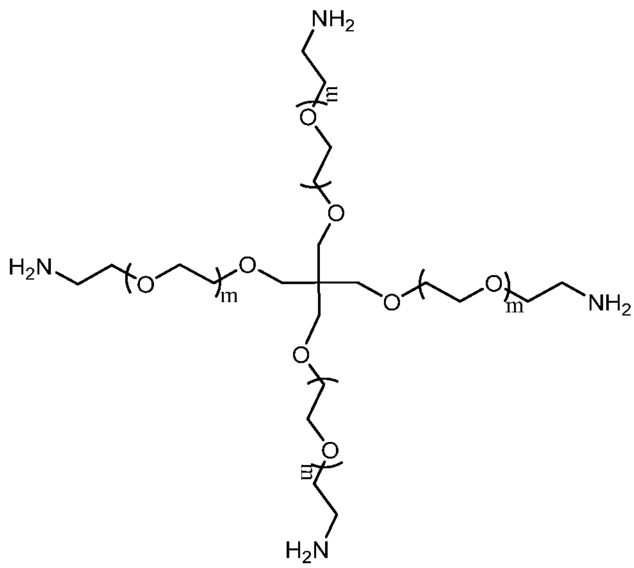

(II),

and

a component G comprising trilysine;

wherein m and n are each a natural number; a molecular weight of the polyethylene glycol derivative I is 2,000-20,000, preferably 5,000-15,000, and more preferably 8,000-12,000; a molecular weight of the polyethylene glycol derivative II is 2,000-20,000, preferably 5,000-15,000, and more preferably 8,000-12,000.

Optionally, any one of the component E, the component F, and the component G is a powder component or a liquid component.

A fifth aspect of the present disclosure provides a kit for tissue closure, characterized in that the kit includes the composition according to the fourth aspect.

Effects

In some embodiments, the present disclosure provides a medical tissue adhesive that is formed by bonding a polyethylene glycol derivative I with a polyethylene glycol derivative II and a trilysine respectively and that has a three-dimensional net-shaped gel framework structure. The crosslinking between the polyethylene glycol derivative I, polyethylene glycol derivative II and trilysine makes the crosslinking network in the gel framework denser and the intermolecular interaction stronger, and enables the tissue adhesive to have higher tissue adhesion strength and mechanical strength. Moreover, the medical tissue adhesive has reduced swelling rate, high biocompatibility, and biodegradability, can effectively adhere to tissues, close wounds, reduce compression on nerves and other tissues, and can realize wound closure, tissue repair, and occlusion of postoperative blood, tissue fluids, and other liquids. It is therefore of clinical significance and carries broad use prospects.

In some embodiments, adjusting the mass ratio between the polyethylene glycol derivative I, the polyethylene glycol derivative II and trilysine enables the medical tissue adhesive provided by the present disclosure to have a gel framework higher in crosslinking density, have stronger adhesion strength and favorable mechanical properties, and satisfactorily match mechanical properties of tissues. The medical tissue adhesive in the present disclosure can favorably adhere to different tissues such as stomach, small intestine, skin, heart, lungs, and liver, keeps sticky and elastic in liquid environments, and can meet different biomedical requirements such as wound closure, liquid occlusion, and postoperative hemostasis.

In some embodiments, the preparation method provided by the present disclosure is simple in step and easy to carry out, uses raw materials low in cost and abundant in source, and is suitable for large-scale industrial mass production.

In some embodiments, the composition for preparing a medical tissue adhesive that is provided by the present disclosure is easy to use and carry out because it can gel and adhere to a tissue in a short time through the mixture of different components.

## BRIEF DESCRIPTION OF THE DRAWINGS

Fig. 1 is a schematic diagram of how the medical tissue adhesive forms and how it adheres to a tissue and arrests bleeding.
Fig. 2 shows results of biocompatibility testing of the medical tissue adhesive on L929 cells, with a scale of 10 μm.
Fig. 3 shows results of testing of trilysine for its purity and chemical structure.
Fig. 4 shows effects of the concentrations of the polyethylene glycol derivatives on the stress-strain curve, modulus of elasticity, and adhesion strength of the medical tissue adhesive.
Fig. 5 shows results of testing of the medical tissue adhesive for its strength of adhesion to, its shear strength against, and interface toughness on different tissues.
Fig. 6 shows effects of the concentrations of the polyethylene glycol derivatives on the shear force and shear strength of the medical tissue adhesive.
Fig. 7 shows effects of the concentrations of the polyethylene glycol derivatives on the peel force and interface toughness of the medical tissue adhesive.
Fig. 8 shows an animal experiment on liver surface hemostasis using the medical tissue adhesive of the present disclosure.
Fig. 9 shows *in vitro* degradation characteristics of the medical tissue adhesive of the present disclosure.

## DETAILED DESCRIPTION

Various exemplary embodiments, features, and aspects of the present disclosure will hereinafter be described in detail. The word "exemplary" used herein means "serving as an example, embodiment, or illustration." Any embodiment illustrated herein as "exemplary" is not necessarily construed as superior to other embodiments.

The embodiments given below include numerous details in order to better explain the present disclosure. It should be understandable to one skilled in the art that the present disclosure can still be implemented without some of the details. In other embodiments, methods, means, apparatus, and steps that are well known to one skilled in the art are not described in detail in order to highlight the points of the present disclosure.

The units of measurement used herein are all those under the International System of Units unless otherwise specified, and the values and numerical ranges in the present disclosure should be understood to include systematic errors inevitable in industrial production.

The symbol "% (m/v)" herein denotes a percent concentration by mass unless otherwise specified and means the mass of a component in a solvent per unit volume. A percent concentration by mass is calculated herein using "mL" as the unit of a volume and "g' as the unit of a mass. For example, in the case of 0.002 g of a component added to 1 mL of a solvent, the percent concentration by mass of the component is (0.002 g/1 mL) × 100% = 0.2% (m/v).

The "molecular weight" of a polyethylene glycol derivative herein denotes the weight average molecular weight of the polyethylene glycol derivative unless otherwise specified.

The meaning of the word "may" herein includes both the meaning of carrying out a treatment and the meaning of not carrying out the treatment.

The term "some specific/preferred embodiments," "other specific/preferred embodiments," "embodiments" or the like herein means that specific elements (e.g., features, structures, properties, and/or characteristics) described in connection with those embodiments are included in at least one of the embodiment described herein, and may or may not exist in other embodiments. It should be understood that the elements may be combined in any suitable manner in various embodiments.

A numerical range indicated by "A-B" herein refers to a range including the endpoints "A" and "B."

The term "water" herein includes any suitable water, such as deionized water, distilled water, ion-exchanged water, twice distilled water, high-purity water, and pure water.

The terms "normal temperature" and "room temperature" used herein refers to a temperature of 10-40 °C.

### Medical tissue adhesives

A first aspect of the present disclosure provides a medical tissue adhesive, which has a gel framework formed by bonding a polyethylene glycol derivative I with a polyethylene glycol derivative II and a trilysine respectively;

wherein a structure of the polyethylene glycol derivative I is shown in Formula (I):

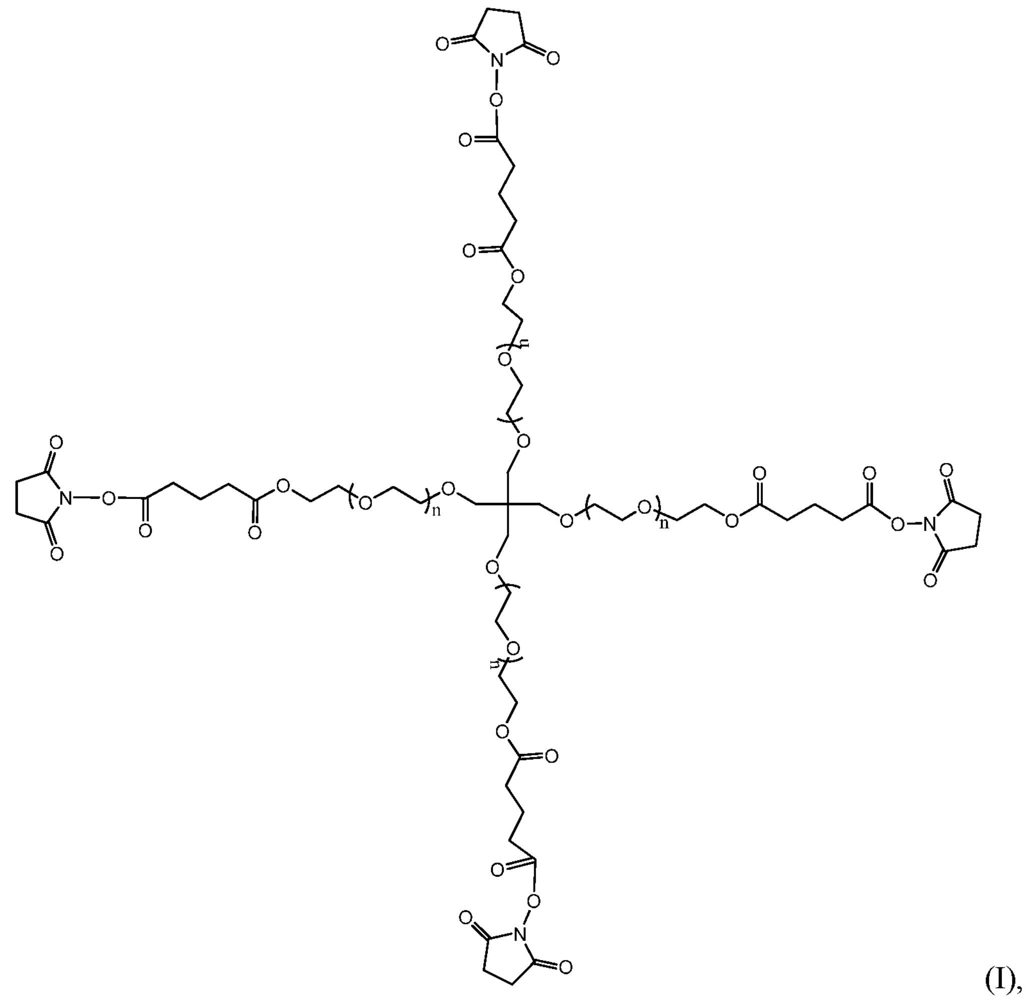

(I),

and

a structure of the polyethylene glycol derivative II is shown in Formula (II):

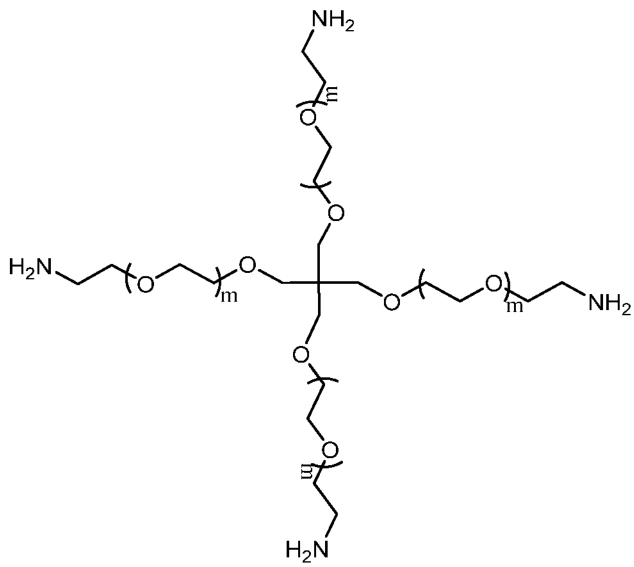

(II),

wherein m and n are each a natural number; a molecular weight of the polyethylene glycol derivative I is 2,000-20,000,

preferably 5,000-15,000, and more preferably 8,000-12,000; a molecular weight of the polyethylene glycol derivative II is 2,000-20,000, preferably 5,000-15,000, and more preferably 8,000-12,000.

The medical hydrogel of the present disclosure is formed by bonding a polyethylene glycol derivative I (4-arm polyethylene glycol-succinimidyl glutarate ester, PEG-SG) represented by Formula (I) with a polyethylene glycol derivative II (4-arm polyethylene glycol-amine, PEG-$NH_2$) represented by Formula (II) and trilysine (SL) respectively. As shown in Fig. 1, Fig. 1(a) shows the molecular structures of the polyethylene glycol derivative I, the polyethylene glycol derivative II, and trilysine. As shown in Fig. 1(a), the polyethylene glycol derivative I has an active succinimidyl ester group which chemically reacts with the amino group of the polyethylene glycol derivative II to form covalent bonds between them, and thereby forming a network crosslinking structure. Simultaneously, the polyethylene glycol derivative I is covalently bonded with trilysine through amide bonds, making the hydrogel framework have higher crosslinking density. As a result, a gel framework structure formed by covalent crosslinking between the three substances that is shown in Fig. 1(a) comes into being.

Functional groups in medical tissue adhesives can be covalently bonded with biological tissues, and the tissue adhesives thereby adhere to the tissues. The present disclosure discovers that the medical tissue adhesive that is formed by the polyethylene glycol derivative I, the polyethylene glycol derivative II, and trilysine has favorable biocompatibility and degradability, improved adhesion to tissues, and reduced swelling rate. Hence, it can reduce compression on nerves and other tissues, effectively adhere to various tissues, and exhibit different biomedical functions such as wound closure, tissue repair, liquid occlusion, and postoperative hemostasis. It carries broad prospects in clinical applications.

In some embodiments, a molecular weight of the polyethylene glycol derivative I with a structure as shown in Formula (I) is 2,000-20,000, preferably 5,000-15,000, and more preferably 8,000-12,000. Exemplarily, the molecular weight of the polyethylene glycol derivative I is 2,000, 5,000, 8,000, 10,000, 15,000, or 20,000, and a molecular weight falling within a range between any two thereof. In the present disclosure, n in Formula (I) is not particularly limited and can be any natural number. Specifically, n is such a value that enables the molecular weight of the polyethylene glycol derivative I to fall within the range of 2,000-20,000. For example, n is 5-107. More specifically, in the present disclosure, that the molecular weight of the polyethylene glycol derivative I is 2,000-20,000 means that the weight average molecular weight of the polyethylene glycol derivative I is 2,000-20,000, and n in Formula (I) is such a value that enables the weight average molecular weight of the polyethylene glycol derivative I to fall within the range of 2,000-20,000.

In some embodiments, a molecular weight of the polyethylene glycol derivative II with a structure as shown in Formula (II) is 2,000-20,000, preferably 5,000-15,000, and more preferably 8,000-12,000. Exemplarily, the molecular weight of the polyethylene glycol derivative II is 2,000, 5,000, 8,000, 10,000, 15,000, or 20,000, and a molecular weight falling within a range between any two thereof. In the present disclosure, m is not particularly limited and can be any natural number. Specifically, m is such a value that enables the molecular weight of the polyethylene glycol derivative II to fall within the range of 2,000-20,000. For example, m is 10-111. More specifically, in the present disclosure, that the molecular weight of the polyethylene glycol derivative II is 2,000-20,000 means that the weight average molecule weight of the polyethylene glycol derivative II is 2,000-20,000, and m in Formula (II) is such a value that enables the weight average molecule weight of the polyethylene glycol derivative II to fall within the range of 2,000-20,000.

The weight average molecular weight described in the present disclosure may be determined by methods commonly used in the art, for example, MALDI-TOF.

In some embodiments, in order for the medical hydrogel to have a higher degree of crosslinking in the gel framework and adhere to a tissue stronger, the mass ratio of the polyethylene glycol derivative I to the polyethylene glycol derivative II is 1: (0.8-1.2), preferably 1: (0.9-1.1), and more preferably 1: (0.95-1.05). Exemplarily, the mass ratio of the polyethylene glycol derivative I to the polyethylene glycol derivative II is 1: 0.8, 1: 0.9, 1: 1, 1: 1.1, or 1: 1.2, and a value falling within a range between any two thereof.

In some embodiments, the mass ratio of the polyethylene glycol derivative I to trilysine is 1: (0.003-0.1), preferably 1: (0.005-0.05), and more preferably 1: (0.01-0.02). Exemplarily, the mass ratio of the polyethylene glycol derivative I to trilysine is 1: 0.005, 1: 0.01, 1: 0.02, 1: 0.04 or 1: 0.05, and a value falling within a range between any two thereof. When the mass ratio of the polyethylene glycol derivative I to trilysine falls within the above ranges, the tissue adhesive can be effectively formed after being fully mixed the components. If the mass ratio is lower than the above ranges, the resulting tissue adhesive will have too high a degree of swelling and thus may compress tissues after implantation. If the mass ratio is higher than the above ranges, the hydrogel will have too high a degree of network crosslinking, and the hydrogel will not degrade satisfactorily after implantation.

In some embodiments, the medical tissue adhesive of the present disclosure is compared with an existing absorbable *dura mater* sealant. The absorbable *dura mater* sealant is formed by crosslinking between 4-arm polyethylene glycol-hydroxysuccinimidyl glutarate ester, polyethyleneimine, and trilysine. Compared with the absorbable *dura mater* sealant, the medical tissue adhesive of the present disclosure has improved adhesion to tissues and significantly improved shear strength and interface toughness, indicating that bonding between the polyethylene glycol derivative I, the polyethylene glycol derivative II, and trilysine in the present disclosure results in synergistically enhanced adhesion to tissues

and mechanical properties. The absorbable *dura mater* sealant needs 8-12 seconds to gel, has a swelling rate of not higher than 185%, and needs 4-8 weeks to degrade. However, the medical tissue adhesive in the present disclosure needs less time (not more than 5 seconds) to gel and more time (not less than 40 days) to degrade and has a lower swelling (not higher than 145%).

In some embodiments, the medical tissue adhesive of the present disclosure is compared with an existing tissue sealant. The tissue sealant comprises a 4-arm polyethylene glycol succinimidyl ester and 4-arm polyethylene glycol-thiol as main components and satisfactorily adheres to tissues. Compared with the tissue sealant, the medical tissue adhesive in the present disclosure has a significantly reduced swelling rate, indicating that bonding between the polyethylene glycol derivative I, the polyethylene glycol derivative II, and the trilysine in the present disclosure improves the adhesive's mechanical properties and adhesion to tissues while reducing the swelling and thereby reducing the compression on nerves and other tissues and being applicable to tissue wounds in limited spaces.

In some embodiments, the medical tissue adhesive is formed at tissue wounds, and mixing the polyethylene glycol derivative I with the polyethylene glycol derivative II and trilysine can create a gel in a short time and thereby produce the effect of adhesion to tissues. The tissue adhesive can adhere to tissues in wet environments, and has high adhesion strength, satisfactory mechanical properties, and a reduced swelling rate. Therefore, it has the functions of tissue repair and hemostasis due to being capable of wound closure and occlusion of blood, tissue fluids, and other liquids.

The medical tissue adhesive in the present disclosure is biocompatible because it is degraded and absorbed *in vivo* and finally metabolized and excreted from the body. Moreover, it avoids the limitations of biological factor-derived adhesives, e.g., the potential immunogenicity of fibrin-based adhesives and the limited activity of the coagulation cascade reaction in some patients. That is, it has favorable biocompatibility and biodegradability.

Methods of preparing medical tissue adhesives

A second aspect of the present disclosure provides a method of preparing a medical tissue adhesive, which comprises:

a step of preparing a solution A comprising a polyethylene glycol derivative I;
a step of preparing a solution B comprising a polyethylene glycol derivative II and trilysine; and
a step of mixing the solution A and the solution B, to obtain the medical tissue adhesive.

In some embodiments, the solution A and the solution B are mixed directly at a tissue wound. Exemplarily, as shown in Fig. 1(b), when the solution A and the solution B are mixed at the time of being simultaneously pressed out, the polyethylene glycol derivative I is cross-linked with the polyethylene glycol derivative II and trilysine. The instantaneous gelling creates a tissue adhesive capable of adhesion. Once formed, the medical tissue adhesive adheres to a defective blood vessel. The amino groups in vascular tissue interact electrostatically and chemically with medical tissue adhesive, forming an adhesive force that achieves the sealing of the wound and playing a hemostatic role by way of physical occlusion. The medical tissue adhesive is satisfactorily biocompatible and free of potential immunogenicity and anticoagulant problems. As the surface of the wound recovers, the medical tissue adhesive gradually degrades and is finally metabolized and excreted from the body.

In the present disclosure, the solution A may be obtained by dissolving the polyethylene glycol derivative I in a solvent suitable for administration *in vivo* or to surface of a body. Exemplarily, the solvent for dissolving the polyethylene glycol derivative I is a phosphate buffer, water, or a different type of solvent in the art. In some embodiments, the solution A is formed by dissolving the polyethylene glycol derivative I in a phosphate buffer. The phosphate buffer is neutral, e.g., having a pH of 7.0-7.4.

In the present disclosure, the solution B may be prepared in any one of the following ways:

In some embodiments, a solution comprising the polyethylene glycol derivative II and a solution comprising trilysine are prepared respectively, and the solution B is obtained by mixing the two solutions.

In some embodiments, a solution comprising the polyethylene glycol derivative II is prepared, and the solution B is obtained by adding trilysine to the solution comprising the polyethylene glycol derivative II.

In some embodiments, a solution comprising trilysine is prepared, and the solution B is obtained by adding the polyethylene glycol derivative II to the solution comprising trilysine.

In some embodiments, the solution B is obtained by mixing the polyethylene glycol derivative II and trilysine and adding them to a solvent.

The solvent for dissolving the polyethylene glycol derivative II and/or trilysine may be a phosphate buffer, water, or a different type of solvent in the art. In some embodiments, the solution B comprising the polyethylene glycol derivative II and trilysine is a phosphate buffer comprising the polyethylene glycol derivative II and trilysine. The phosphate buffer is neutral, e.g., having a pH of 7.0-7.4.

In some more specific embodiments, the solution B is obtained by mixing trilysine and the polyethylene glycol

derivative II and dissolving them in a phosphate buffer. Alternatively, the solution B is obtained by dissolving the polyethylene glycol derivative II in a phosphate buffer, and adding trilysine to the solution. Alternatively, the solution B is obtained by dissolving trilysine in a phosphate buffer, and adding the polyethylene glycol derivative II to the solution. Alternatively, the solution B is obtained by dissolving trilysine in a phosphate buffer, dissolving the polyethylene glycol derivative II in a phosphate buffer, and mixing the two solutions.

In some embodiments, the polyethylene glycol derivative I is present in the solution A in a percent concentration by mass of 10-30% (m/v), and preferably 18-22% (m/v). Exemplarily, the polyethylene glycol derivative I is present in the solution A in a percent concentration by mass of 12% (m/v), 14% (m/v), 16% (m/v), 18% (m/v), 20% (m/v), 22% (m/v), 24% (m/v), 26% (m/v), or 28% (m/v), and in a percent concentration by mass of a value falling a range between any two thereof.

In some embodiments, the polyethylene glycol derivative II is present in the solution B in a percent concentration by mass of 10-30% (m/v), and preferably 18-22% (m/v). Exemplarily, the polyethylene glycol derivative I is present in the solution A in a percent concentration by mass of 12% (m/v), 14% (m/v), 16% (m/v), 18% (m/v), 20% (m/v), 22% (m/v), 24% (m/v), 26% (m/v), or 28% (m/v), and in a percent concentration by mass of a value falling a range between any two thereof.

Increasing the concentration by mass of the polyethylene glycol derivative I and/or that of the polyethylene glycol derivative II to a certain extent enables the medical tissue adhesive to have higher mechanical strength and be stronger in adhesion to tissues. However, it is necessary to consider that the medical tissue adhesive needs to have mechanical properties matching those of a primary tissue. When the polyethylene glycol derivative I and the polyethylene glycol derivative II are present in a concentration by mass that falls within the above ranges, the medical tissue adhesive has favorable mechanical properties and strong adhesion and matches a primary tissue.

In some embodiments, trilysine is present in the solution B in a percent concentration by mass of 0.06%-2% (m/v), preferably 0.1%-1% (m/v), and more preferably 0.2%-0.4% (m/v). Exemplarily, trilysine is present in the solution B in a percent concentration by mass of 0.08% (m/v), 0.1% (m/v), 0.15% (m/v), 0.2% (m/v), 0.25% (m/v), 0.3% (m/v), 0.35% (m/v), 0.4% (m/v), 0.5% (m/v), 0.7% (m/v), 0.9% (m/v), 1.1% (m/v), 1.5% (m/v), 1.8% (m/v), and in a percent concentration by mass of a value falling within a range between any two of them. When trilysine is present in the solution B in a concentration by mass that falls within the above ranges, the medical tissue adhesive has improved mechanical strength and a reduced swelling rate, is suitable for tissue adhesion and liquid occlusion, and is biocompatible and degradable.

In the present disclosure, the solution A and the solution B may be mixed directly in the same tissue region; alternatively, it may be applied to two separate tissue regions respectively, and mixed in the course of attaching the two tissue regions.

The term "the same tissue region" in the present disclosure may be located in a tissue at any location. For the convenience of description, two tissue regions to be bound are hereinafter referred to as "a/the first tissue region" and "a/the second tissue region."

In some embodiments, the solution A and the solution B are mixed directly in the same tissue region. Exemplarily, the solution A comprising the polyethylene glycol derivative I and the solution B comprising the polyethylene glycol derivative II and trilysine are formulated in the two separate lines of a dual-chamber syringe. The solution A and the solution B are simultaneously applied to the first tissue region by the dual-chamber syringe, and they are mixed while they are being pressed out. The interaction between the polyethylene glycol derivative I, the polyethylene glycol derivative II, and trilysine leads to the formation of a hydrogel capable of adhesion to tissues. At this time, attaching a side of the first tissue region where the medical tissue adhesive is applied to a second tissue region where the medical tissue adhesive is not applied makes the first tissue region strongly adheres to the second tissue region.

In some embodiments, the solution A and the solution B are applied to two tissue regions where adhesion is required, respectively. Exemplarily, the solution A is applied to a first tissue region, the solution B is applied to a second tissue region, and a side of the first tissue region where the solution A is applied is attached to a side of the second tissue region where the solution B is applied. The solution A and the solution B, while coming into contact with each other, are mixed and create a medical tissue adhesive that enables the first tissue region and the second tissue region to adhere to each other.

Compositions for preparing medical tissue adhesives

A third aspect of the present disclosure provides a composition for preparing a medical tissue adhesive, which comprises:

a component E comprising a polyethylene glycol derivative I with a structure as shown in Formula (I):

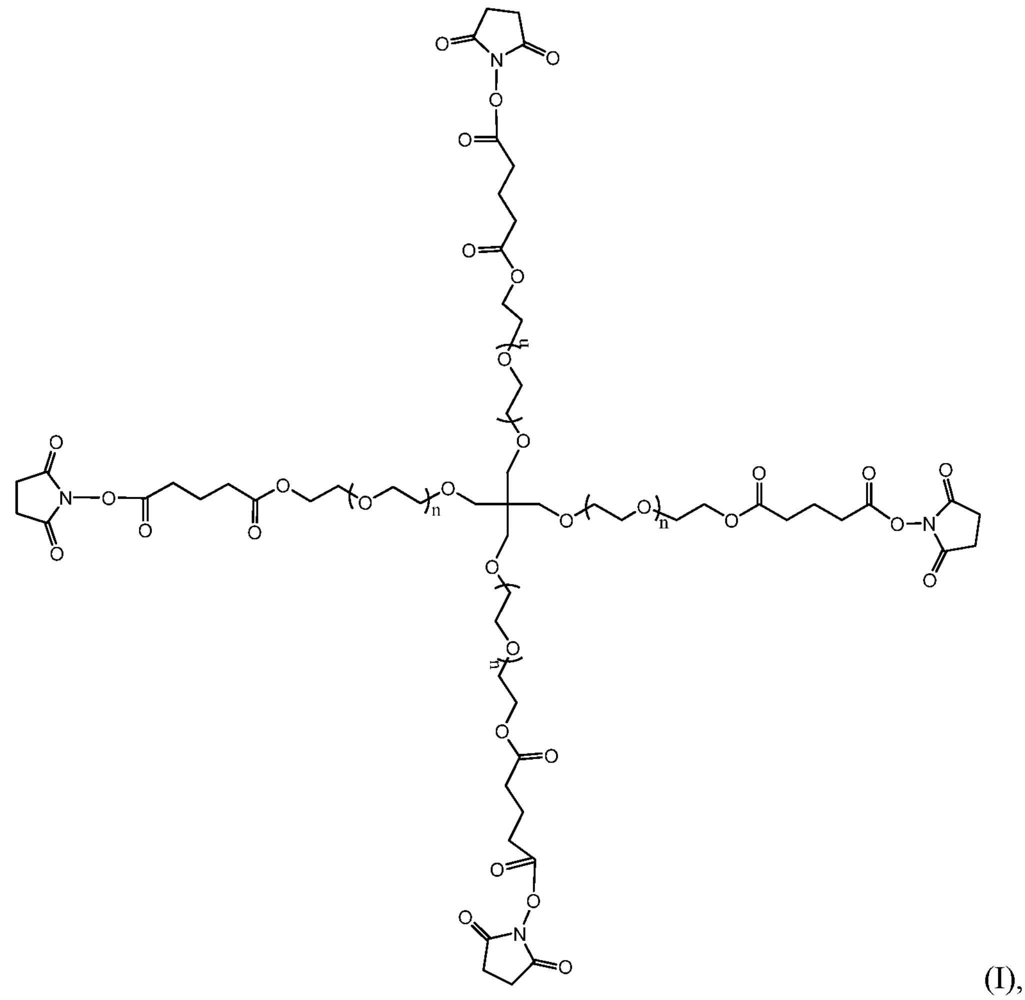

(I),

a component F comprising a polyethylene glycol derivative II with a structure as shown in Formula (II):

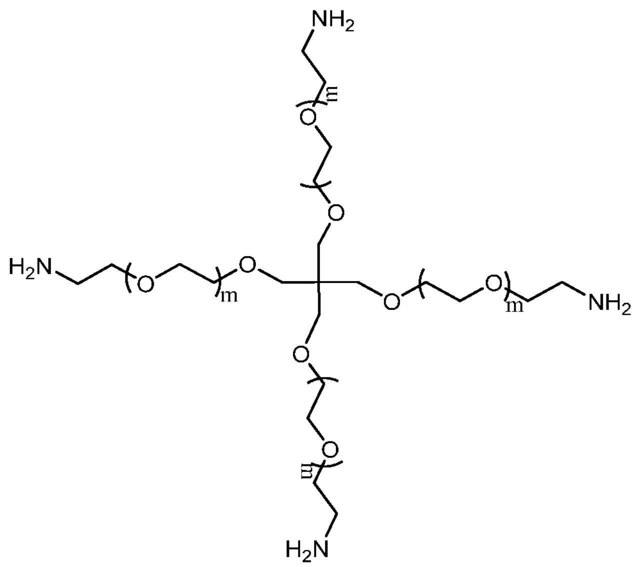

(II),

and

a component G comprising trilysine;

wherein m and n are each a natural number; a molecular weight of the polyethylene glycol derivative I is 2,000-20,000,

preferably 5,000-15,000, and more preferably 8,000-12,000; a molecular weight of the polyethylene glycol derivative II is 2,000-20,000, preferably 5,000-15,000, and more preferably 8,000-12,000. The preparation kit in the present disclosure can gel within 5 s by making use of the three components E, F and G, and the composition, upon applied to a tissue surface, has the function of adhesion to tissues and thus generates a medical tissue adhesive having favorable mechanical properties, strong tissue adhesion, high biocompatibility, and a low swelling rate. Mixing the components E, F and G on a wound surface can instantly generate a medical hydrogel capable of adhesion and thus capable of meeting different biomedical needs such as wound closure, tissue adhesion, liquid occlusion, and postoperative hemostasis.

In some embodiments, the component E is a solid reagent. For example, the component E is a dry powder of the polyethylene glycol derivative I. In some embodiments, the component E is a liquid reagent. For example, the component E is obtained by dissolving the polyethylene glycol derivative I in a solvent. The solvent for dissolving the polyethylene glycol derivative I may be a PBS or a solvent type useful in the art.

In some embodiments, the component F is a solid reagent. For example, the component F is a dry powder of the polyethylene glycol derivative II. In some embodiments, the component F is a liquid reagent. For example, the component F is obtained by dissolving the polyethylene glycol derivative II in a solvent. The solvent for dissolving the polyethylene glycol derivative II may be PBS or a solvent type useful in the art.

In some embodiments, the component G is a solid reagent. For example, the component G is a dry powder of trilysine. In some embodiments, the component G is a liquid reagent. For example, the component G is obtained by dissolving trilysine in a solvent. The solvent for dissolving the trilysine may be PBS or a solvent type useful in the art.

In some embodiments, the component F and the component G may also be present as a mixed reagent. For example, a mixed dry powder of the polyethylene glycol derivative II and trilysine. Alternatively, it is a mixed solution in which the polyethylene glycol derivative II and trilysine are both dissolved.

Exemplarily, the component E, the component F, and the component G are present in a composition for preparing a medical tissue adhesive in such a manner that the component E is a separate solid or liquid reagent (e.g., a separately packaged dry powder or solution), the component F is a separate solid or liquid reagent (e.g., a separately packaged dry powder or solution), and the component G is a separate solid or liquid reagent (e.g., a separately packaged dry powder or solution).

Alternatively, the component E, the component F, and the component G are present in the composition in such a manner that the component E is a separate solid or liquid reagent (e.g., a separately packaged dry powder or solution), and the component F and the component G are a mixed solid or liquid reagent (e.g., a separately packaged dry powder or solution of the mixed component E and the component G).

<u>Kits for tissue closure</u>

A fourth aspect of the present disclosure provides a kit for tissue closure, which comprises the composition for preparing a medical tissue adhesive according to the third aspect. The kit in the present disclosure can quickly generate, on the surface of, or inside a tissue, a medical tissue adhesive that has high biocompatibility, degradability, high mechanical strength, and a low swelling rate and that strongly adheres to different tissues such as stomach, small intestine, skin, heart, lungs, and liver and thereby effectively plays the roles of wound closure, liquid occlusion, tissue repair, and the like.

In some embodiments, the kit also includes an instruction manual that reads as follows:

(1) When the kit is used to prepare a medical hydrogel, the mass ratio of the polyethylene glycol derivative I to the polyethylene glycol derivative II is 1: (0.8-1.2), preferably 1: (0.9-1.1), and more preferably 1: (0.95-1.05).
(2) When the kit is used to prepare a medical hydrogel, the mass ratio of the polyethylene glycol derivative I to trilysine is 1: (0.003-0.1), preferably 1: (0.005-0.05), and more preferably 1: (0.01-0.02).

In some embodiments, the kit also includes an instruction manual that reads as follows:

(1) Preparing a solution A comprising the polyethylene glycol derivative II using the component E;
(2) Preparing a solution B comprising the polyethylene glycol derivative II and trilysine using the component F and the component G; and
(3) Mixing the solution A and the solution B, which results in a medical hydrogel.

In some more specific embodiments, the instruction manual reads as follows:
A solution B is prepared from the polyethylene glycol derivative II and trilysine, and trilysine is present in the solution B in a percent concentration by mass of 0.06%-2% (m/v), preferably 0.1%-1% (m/v), and more preferably 0.2%-0.4%

(m/v); the polyethylene glycol derivative II is present in the solution B in a percent concentration by mass of 10-30% (m/v), and preferably 18-22% (m/v).

In some more specific embodiments, the instruction manual reads as follows:

A solution A is prepared from the polyethylene glycol derivative I, and the polyethylene glycol derivative I is present in the solution A in a percent concentration by mass of 10-30% (m/v), and preferably 18-22% (m/v).

In some embodiments, a dual-chamber syringe is also included in the kit. The solution A and the solution B are mixed well while they are being pressed out by a customized nozzle of the dual-chamber syringe, and change into a gel capable of adhesion to tissues in a short time.

Use of medical tissue adhesives

A fourth aspect of the present disclosure provides use of the medical tissue adhesive. In some embodiments, the tissue adhesive acts as or is used to prepare an adhesive for endoscope closure. In some embodiments, the tissue adhesive acts as or is used to prepare an adhesive for blood vessel closure. In some embodiments, the tissue adhesive acts as or is used to prepare an adhesive for surgery closure. In some embodiments, the tissue adhesive acts as or is used to prepare an adhesive for cerebrospinal fluid closure.

The medical tissue adhesive in the present disclosure can effectively adhere to tissues, thereby quickly arresting bleeding and effectively closing wounds and helping to heal wounds. Therefore, it can be applied to a large number of scenarios in gastrointestinal surgery, hand and foot surgery, neurosurgery, and other fields.

## Examples

Embodiments of the present disclosure will be described in detail by way of examples, but it would be understandable to one skilled in the art that the examples are only intended to illustrate the present disclosure and should not be taken as limitations on the scope of the present disclosure. In the case where the examples do not indicate a specific condition, an ordinary condition or a condition recommended by manufacturers was used. In the case where the examples do not indicate the manufacturer of a reagent or instrument used therein, the reagent or instrument was an ordinary product commercially available.

## Example 1

In this example, trilysine (SL), 4-arm polyethylene glycol-succinimidyl glutarate ester (PEG-SG), and 4-arm polyethylene glycol-amine (PEG-$NH_2$) were used as the raw material to prepare a medical tissue adhesive. The 4-arm polyethylene glycol-succinimidyl glutarate ester has a structure of Formula (I) and a molecular weight of 10,000. The 4-arm polyethylene glycol-amine has a structure of Formula (II) and a molecular weight of 10,000.

The medical tissue adhesive was prepared by the following steps:

(1) Preparing a solution A by dissolving 200 mg of the 4-arm polyethylene glycol-succinimidyl glutarate ester in 1 mL of a neutral phosphate buffer (PBS), the percent concentration by mass of the 4-arm polyethylene glycol-succinimidyl glutarate ester in the solution A being 20% (m/v);
(2) Preparing a solution B by dissolving 200 mg of the 4-arm polyethylene glycol-amine and 2 mg of trilysine in 1 mL of a neutral phosphate buffer, the percent concentration by mass of the 4-arm polyethylene glycol-amine being 20% (m/v), and that of trilysine being 0.2% (m/v); and
(3) Obtaining the medical tissue adhesive by pressing out the two solutions simultaneously using a dual-chamber syringe.

## Example 2

In this example, trilysine, 4-arm polyethylene glycol-succinimidyl glutarate ester, and 4-arm polyethylene glycol-amine were used as the raw material to prepare a medical tissue adhesive. The 4-arm polyethylene glycol-succinimidyl glutarate ester has a structure of Formula (I) and a molecular weight of 5,000. The 4-arm polyethylene glycol-amine has a structure of Formula (II) and a molecular weight of 5,000.

The medical tissue adhesive was prepared by the following steps:

(1) Preparing a solution A by dissolving 300 mg of the 4-arm polyethylene glycol-succinimidyl glutarate ester in 1 mL of a neutral phosphate buffer, the percent concentration by mass of the 4-arm polyethylene glycol-succinimidyl glutarate ester in the solution A being 30% (m/v);
(2) Preparing a solution B by dissolving 300 mg of the 4-arm polyethylene glycol-amine and 1 mg of trilysine in 1

mL of a neutral phosphate buffer, the percent concentration by mass of the 4-arm polyethylene glycol-amine being 30% (m/v), and that of trilysine being 0.1% (m/v); and

(3) Obtaining the medical tissue adhesive by pressing out the two solutions simultaneously using a dual-chamber syringe.

**Example 3**

In this example, trilysine, 4-arm polyethylene glycol-succinimidyl glutarate ester, and 4-arm polyethylene glycol-amine were used as the raw material to prepare a medical tissue adhesive. The 4-arm polyethylene glycol-succinimidyl glutarate ester has a structure of Formula (I) and a molecular weight of 20,000. The 4-arm polyethylene glycol-amine has a structure of Formula (II) and a molecular weight of 20,000.

The medical tissue adhesive was prepared by the following steps:

(1) Preparing a solution A by dissolving 100 mg of the 4-arm polyethylene glycol-succinimidyl glutarate ester in 1 mL of a neutral phosphate buffer, the percent concentration by mass of the 4-arm polyethylene glycol-succinimidyl glutarate ester in the solution A being 10% (m/v);

(2) Preparing a solution B by dissolving 100 mg of the 4-arm polyethylene glycol-amine and 3 mg of trilysine in 1 mL of a neutral phosphate buffer, the percent concentration by mass of the 4-arm polyethylene glycol-amine being 10% (m/v), and that of trilysine being 0.3% (m/v); and

(3) Obtaining the medical tissue adhesive by pressing out the two solutions simultaneously using a dual-chamber syringe.

**Example 4**

In this example, trilysine, 4-arm polyethylene glycol-succinimidyl glutarate ester, and 4-arm polyethylene glycol-amine were used as the raw material to prepare a medical tissue adhesive. The 4-arm polyethylene glycol-succinimidyl glutarate ester (4 arm-PEG-SG) has a structure of Formula (I) and a molecular weight of 10,000. The 4-arm polyethylene glycol-amine (4 arm-PEG-$NH_2$) has a structure of Formula (II) and a molecular weight of 15,000.

The medical tissue adhesive was prepared by the following steps:

(1) Preparing a solution B by completely dissolving 200 mg of the 4-arm polyethylene glycol-succinimidyl glutarate ester (4 arm-PEG-SG) in 1 mL of a PBS having a pH of 7.0-7.4, the percent concentration by mass of the 4 arm-PEG-SG in the solution B being 20% (m/v);

(2) Preparing a solution A by completely dissolving 200 mg of the 4-arm polyethylene glycol-amine and 2 mg of trilysine in 1 mL of a PBS having a pH of 7.0-7.4, the percent concentration by mass of the 4-arm polyethylene glycol-amine being 20% (m/v), and that of trilysine being 0.2% (m/v); and

(3) Obtaining the absorbable medical tissue adhesive by adding the two solutions to a dual-chamber syringe and pressing out the two solutions simultaneously using the dual-chamber syringe.

**Example 5**

In this example, trilysine, 4-arm polyethylene glycol-succinimidyl glutarate ester, and 4-arm polyethylene glycol-amine were used as the raw material to prepare a medical tissue adhesive. The 4-arm polyethylene glycol-succinimidyl glutarate ester has a structure of Formula (I) and a molecular weight of 20,000. The 4-arm polyethylene glycol-amine has a structure of Formula (II) and a molecular weight of 15,000.

The medical tissue adhesive was prepared by the following steps:

(1) Preparing a solution A, namely a neutral phosphate solution of the 4-arm polyethylene glycol-succinimidyl glutarate ester (1 mL, 100 mg/mL);

(3) Sucking the solutions A and B into a 2.5 mL syringe, respectively, and installing the syringes into a holder of dual-chamber syringes; and

(4) Obtaining the absorbable medical tissue adhesive by pressing out the two solutions simultaneously, which combined at the tissue surface with each other upon being pressed out.

**Example 6**

In this example, trilysine, 4-arm polyethylene glycol-succinimidyl glutarate ester , and 4-arm polyethylene glycol-amine were used as the raw material to prepare a medical tissue adhesive. The 4-arm polyethylene glycol-succinimidyl

glutarate ester has a structure of Formula (I) and a molecular weight of 10,000. The 4-arm polyethylene glycol-amine has a structure of Formula (II) and a molecular weight of 10,000.

The medical tissue adhesive was prepared by the following steps:

(1) Preparing a solution A, namely a neutral phosphate solution of the 4-arm polyethylene glycol-succinimidyl glutarate ester (1 mL, 100 mg/mL);
(2) Preparing a solution B, namely a neutral phosphate solution of the 4-arm polyethylene glycol-amine and trilysine (1 mL, 100 mg/mL for the 4-arm polyethylene glycol-amine and 2 mg/mL for trilysine);
(3) Obtaining the absorbable medical tissue adhesive by applying the Solution A and B to surfaces of different tissues, respectively, and attaching the two surfaces with the solution A and B to each other.

**Property testing**

1. Biocompatibility

The cytotoxicity of an extract of the medical tissue adhesive to L929 cells were tested in accordance with GB/T 16886. A solution comprising the PEG-SG and a solution comprising the $PEG-NH_2$ and trilysine were pressed out simultaneously into a polytetrafluoroethylene mold (a cylindrical mold, 1 cm in diameter and 1 cm in depth) using a dual-chamber syringe. The resulting hydrogel soaked in 1 mL of a medium ($\alpha$-MEM supplemented with 10% fetal bovine serum and 100 IU/mL penicillin and streptomycin) in a cell incubator at 37 °C for 24 hours at a ratio of 0.1 g/mL (i.e., 0.1 g of the hydrogel in 1 mL of the medium). Next, the supernatant of the medium was extracted. At this point, an ordinary medium in which L929 cells had been inoculated for 24 hours was replaced with the conditioned medium in which the tissue adhesive had soaked, and the cell culture proceeded for 24 hours. Then, the cells were incubated in 10% CCK-8 reagent for 2 hours, and the optical density (OD) of the supernatant at 450 nm was measured by a microplate reader. The cell viability was calculated by this equation:

$$\text{Cell viability (\%)} = (OD_{\text{tissue adhesive}} - OD_{\text{blank}})/(OD_{\text{culture plate}} - OD_{\text{blank}}),$$

where $OD_{blank}$ refers to the optical density of CCK-8 reagent, and $OD_{\text{culture plate}}$ refers to the optical density of L929 cells in the ordinary medium. At this time, the L929 cells were stained with the fluorescent dye calcein-AM/PI for 20 min. The cell state was observed under a laser confocal microscope (488 nm for calcein excitation and 515 nm for emission; 530 nm for PI excitation and 580 nm for emission). Fig. 2 shows results of biocompatibility testing of the medical tissue adhesive on L929 cells, with a scale of 10 $\mu$m. The medical tissue adhesive was prepared using 20% (m/v) PEG-SG, 20% (m/v) $PEG-NH_2$, and 0.2% (m/v) SL. As can be seen from FIG. 2, the medical tissue adhesive prepared in the present disclosure has favorable cell compatibility, characterized by a cell viability of 80% or more.

2. Purity and chemical structure of trilysine

HPLC and NMR were used to detect the purity and chemical structure of trilysine for preparing the medical tissue adhesive. Fig. 3 shows results of testing of trilysine for its purity and chemical structure. As shown in Fig. 3, trilysine used to prepare the medical tissue adhesive had a purity of 99.25%, and it had a chemical structure consistent with the theoretical structure of trilysine.

3. Gelling time

Dissolve 4-arm polyethylene glycol-succinimidyl glutarate ester completely in a neutral phosphate solution, and dissolve 4-arm polyethylene glycol-amine and trilysine (SL) completely in a neutral phosphate solution. Load a dual-chamber syringe with the two solutions. Press out a total of 2 mL of the two solutions quickly at the same time into a vial. Turn the vial upside down and determine whether gelling was completed by observing whether the liquid therein could flow. Record the time that the liquid in the vial needed to completely gel as gelling time. The gelling time was found to be not more than 5 seconds.

4. Mechanical properties

4.1 Modulus of Elasticity

Fill a customized dumbbell-shaped polytetrafluoroethylene mold (with an effective stretch length of 2 cm, a

width of 0.5 cm, and a depth of 0.5 cm) with the medical tissue adhesive. Make sure the mold was full and no obvious bubbles were in the medical tissue adhesive. Wait until the gelling was completed (about 10 minutes). Take the gel out of the mold and use it as the sample for the detection of modulus of elasticity. Fix both ends of the medical adhesive and make sure there was an effective length of 2 cm before the detection. Input parameters of the sample for detection (including a distance of 2 cm, a length of 2 cm, a thickness of 0.5 cm, and a width of 0.5 cm) into the instrument. Reset the instrument to remove irrelevant parameters (including force, displacement, and time) before the detection. Stretch the sample at a stretch speed of 5 mm/min at room temperature until it fractured. Record the data of stress-strain, draw a stress-strain curve, and analyze the curve. Use the slope of a segment of the curve at the elastic deformation stage as the modulus of elasticity of the medical tissue adhesive.

4.2 Adhesion strength

Detect the adhesion strength by referring to ASTM F2258 and using an electronic universal testing machine. Remove surface fat from a piece of pig's back skin newly purchased by rinsing it and letting it soak in a lipase solution having a concentration of 2 wt.% (with a ratio of 100 g of the pigskin in 500 mL of the lipase solution) three times, 30 minutes for each time. The soaking was companied by sonication at a power of 240 W. Cut the treated pigskin into squares of 2 cm $\times$ 2 cm. Stick two squares to two molds for adhesion strength testing by attaching the smoother side of each of the square to the mold with 502 glue. Wait for 1 minute to let the squares firmly attach to the molds. Then, spray 100 $\mu$L of the tissue adhesive on the surface of each of the squares, and attach the two squares to each other quickly. Let them stay at room temperature for 60 minutes to guarantee firm attachment. Pull them at a speed of 1 mm/min at room temperature until the two squares of pigskin were separated. Record the data of force-displacement, draw a force-displacement curve, and analyze the curve. Record the maximum adhesion force ($F_{max}$). Calculate the adhesion strength of the medical tissue adhesive by this equation: Adhesion strength (Pa) = $F_{max}$ (N)/stressed area ($m^2$).

4.3 Shear strength and peel strength

Detect the shear strength by referring to ASTM F2255. Subject a piece of fresh pig's back skin to degreasing and cut it into small pieces of 5 cm $\times$ 2.5 cm. Apply 100 $\mu$L of the tissue adhesive evenly on one end of a small piece, with a coating area of 2.5 cm $\times$ 1 cm. Attach both ends of the small piece to each other quickly in an opposite direction (the attachment area being the coating area). Let it stay at room temperature for 30 minutes to guarantee firm attachment. Pull it at a speed of 5 mm/min at room temperature until the two ends were separated. Record the data of force-displacement, draw a force-displacement curve, and analyze the curve. Record the maximum shear force ($F_{max}$). Calculate the shear force of the medical tissue adhesive by this equation: Shear strength (Pa) = $F_{max}$ (N)/stress area ($m^2$).

Detect the peel strength by referring to ATSM F2256. Subject a piece of fresh pig's back skin to degreasing and cut it into small pieces of 5 cm $\times$ 2.5 cm. Apply 100 $\mu$L of the tissue adhesive evenly on one end of a small piece with a coating area of 2.5 cm $\times$ 1 cm. Attach both ends of the small piece to each other quickly in the same direction (the attachment area being the coating area). Let it stay at room temperature for 30 minutes to guarantee firm attachment. Pull it at a speed of 5 mm/min at room temperature until the two ends were separated. Record the data of force-displacement, draw a force-displacement curve, and analyze the curve. Record the maximum peel force ($F_{max}$). Calculate the interface toughness of the medical tissue adhesive by this equation: Interface toughness ($J_M^{-2}$) = 2 $\times$ $F_{max}$/width (m).

Sterilize the medical tissue adhesive by radiation with a dose of 15kGy and 20kGy. The radiation was validated to achieve a 100% sterilization effect. Fig. 4 shows effects of the concentrations of the polyethylene glycol derivatives I and II on the stress-strain curve, modulus of elasticity, and adhesion strength of the medical tissue adhesive. As can be seen from Fig. 4, increasing their concentrations within a certain range significantly improved the mechanical strength and adhesion strength of the medical tissue adhesive. Considering that the medical tissue adhesive needs to have mechanical properties matching those of a primary tissue, the polyethylene glycol derivatives I and II having a percent concentration by mass of 10%-30% (m/v) and trilysine having a percent concentration by mass of 0.06%-2% (m/v) are selected for using to prepare the tissue adhesive.

Fig. 5 shows results of testing of the medical tissue adhesive for its adhesion to different tissues. As can be seen from Fig. 5, the medical tissue adhesive was capable of adhesion to animal tissues including stomach, small intestine, heart, lungs, and liver. Since soft tissues vary in mechanical strength, the tissue adhesive also varied in adhesion strength, but it was generally satisfactory in adhesion strength, shear strength, and interface toughness. Therefore, it can be applied to different tissues for wound hemostasis and air leakage inhibition.

Pigskin was used as an example for the purpose of easy sampling. Fig. 6 shows effects of different methods of preparing the medical tissue adhesive on the gelling time and shear force. The medical tissue adhesive in connection with Fig. 6 was prepared by two methods. Method 1 was applying the solution A and the solution B to two sides of a piece of pigskin, respectively, and attaching them to each other. Method 2 was applying the solutions using a dual-chamber syringe on one side of a piece of pigskin and attaching it to another piece of pigskin. The shear strength of the

adhesive prepared by these two methods was detected in accordance with ASTM F2255. The different preparation methods were proven to have little influence on the medical tissue adhesive.

Fig. 6 shows effects of the concentrations of the polyethylene glycol derivatives on the shear force and adhesion strength of the medical tissue adhesive. Fig. 6(a) shows the shear force of medical tissue adhesives in different concentrations after they were exposed to electron radiation, and Fig. 6(b) shows the shear strength of medical tissue adhesives in different concentrations after they were exposed to electron radiation.

Fig. 7 shows effects of the concentrations of the polyethylene glycol derivatives on the peel force and interface toughness of the medical tissue adhesive. Fig. 7(a) shows the peel force of medical tissue adhesives in different concentrations after they were exposed to electronic radiation, and Fig. 7(b) shows the interface toughness of medical tissue adhesives in different concentrations after they were exposed to electronic radiation.

In the shear, adhesion and peel experiments of medical tissue adhesives with different concentrations, as can be seen from Figs. 6 and 7, medical tissue adhesives in different concentrations did not vary much in shear strength, but they had interface toughness that first increased and then decreased as their concentrations increased, a trend attributed to their modulus of elasticity. In Figs. 4, 6, and 7, SG-10%, SG-20%, and SG-30% indicate that the PEG-SG was in concentrations of 10% (m/v), 20% (m/v), and 30% (m/v), respectively. As also shown in these drawings, the polyethylene glycol derivative I and the polyethylene glycol derivative II were used in the same concentrations, for an exemplary purpose. This should not be interpreted as constraints on the concentration of the polyethylene glycol derivatives.

Fig. 8 shows an animal experiment on liver surface hemostasis using the medical tissue adhesive of the present disclosure. In the experiment, the animals were healthy Bama miniature pigs. As shown in Fig. 8(a), the animal was anesthetized and had its liver exposed. Fig. 8(b) shows a liver defect model. The tissue defect had a depth of 2 mm and was generated by a ring cutter with a diameter of 8 mm, and blood flew from the defect. As shown in Fig. 8(c), the medical tissue adhesive was applied to the defect. Immediately after they were pressed out and applied on the defect, the liquids gelled, thereby closing the wound surface and arresting bleeding (see Fig. 8(d)). As is evident from the experiment, the tissue adhesive effectively adhered to the animal tissue and closed the wound surface, thereby achieving the effects of hemostasis and wound surface isolation.

Fig. 9 shows *in vitro* degradation characteristics of the medical tissue adhesive of the present disclosure. Converted from an *in vitro* degradation curve, the tissue adhesive formed from the polyethylene glycol derivative I, the polyethylene glycol derivative II, and trilysine, after *in vivo* implanted, had its quality reduced by not less than 30% on the 35th day, and had, 42 days later, a residue fewer than 10%. This indicates that the tissue adhesive provided an effective protection for wound healing, and degraded completely after the tissue repair.

After the liquid changes into a hydrogel, the hydrogel will continue to absorb the solution and swell-thereby gaining weight and becoming more bulky-until swelling equilibrium is reached. Thus, the swelling rate of a hydrogel can be calculated on a volume basis or on a weight basis. Generally, it is calculated using the volume and/or weight of a gel at the time of gelling and the volume and/or weight after a certain period of time. In the present disclosure, the swelling rate was the ratio of the weight of the hydrogel at the time of gelling to its weight after swelling equilibrium was reached, and the time interval time was 24 hours.

The above examples only serve to illustrate the present disclosure and are not meant to limit the embodiments of the present disclosure. To one skilled in the art, the above description can be varied or modified in different ways. It is unnecessary herein to give an exhaustive list of all embodiments. Any modifications, equivalent substitutions, and improvements that do not depart from the spirit and principle of the present disclosure shall be deemed to fall within the scope of protection sought by the claims of the present disclosure.

## Claims

**1.** A medical tissue adhesive, wherein the medical tissue adhesive has a gel framework formed by bonding a polyethylene glycol derivative I with a polyethylene glycol derivative II and a trilysine respectively;

wherein a structure of the polyethylene glycol derivative I is shown in Formula (I):

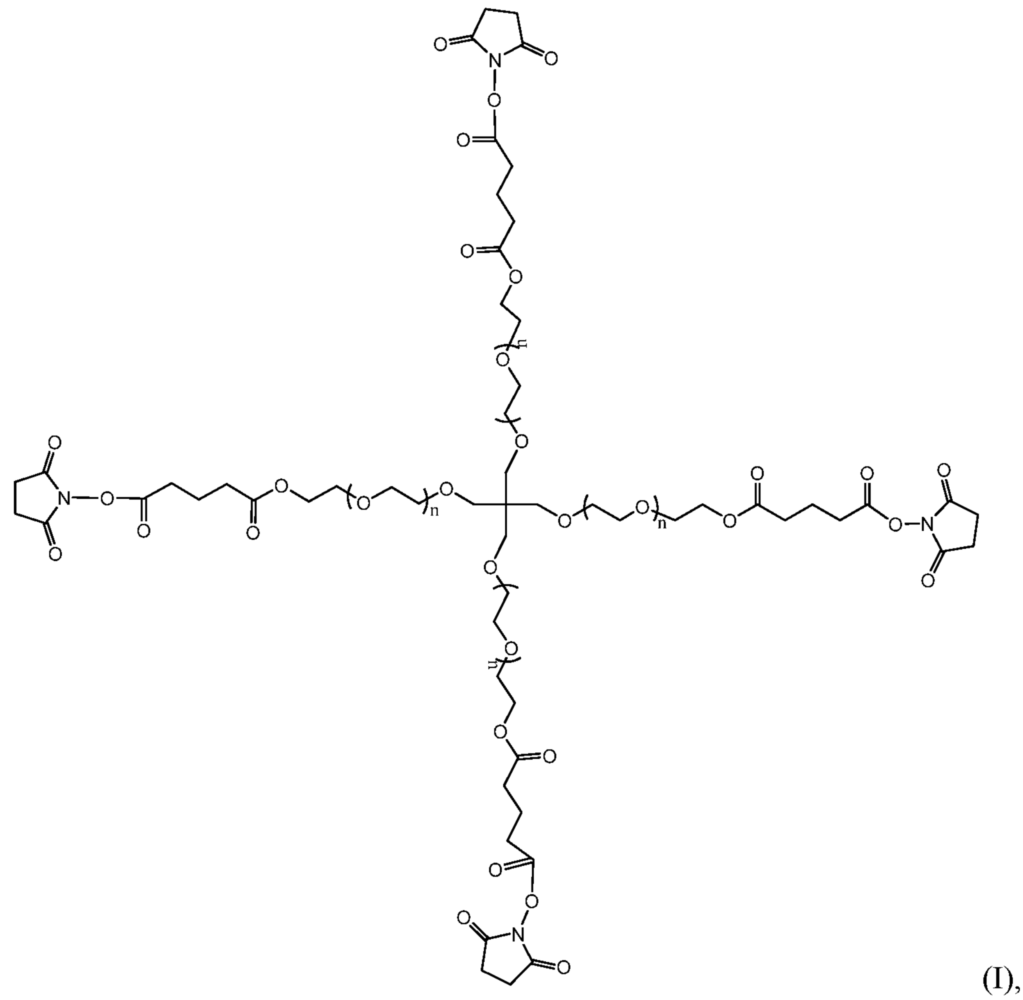

(I),

and

a structure of the polyethylene glycol derivative II is shown in Formula (II):

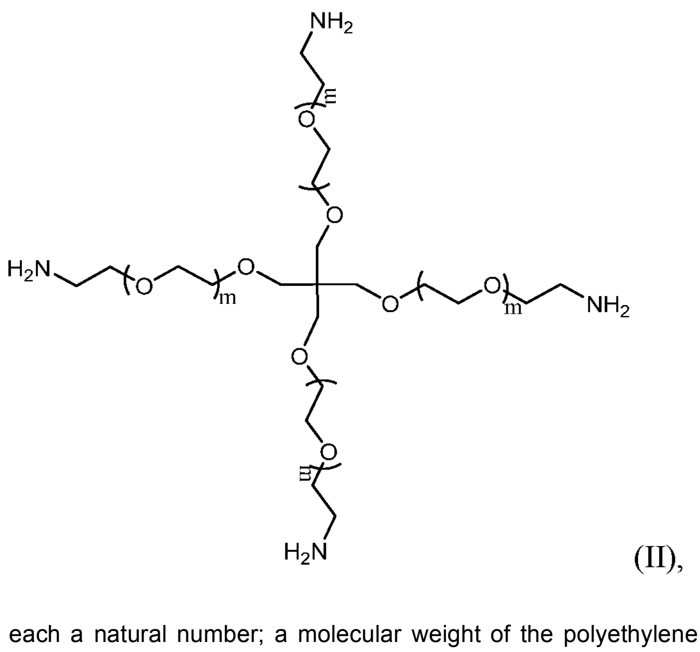

(II),

wherein m and n are each a natural number; a molecular weight of the polyethylene glycol derivative I is 2,000-20,000, preferably 5,000-15,000, and more preferably 8,000-12,000; a molecular weight of the polyethylene glycol derivative II is 2,000-20,000, preferably 5,000-15,000, and more preferably 8,000-12,000.

2. The medical tissue adhesive according to claim 1, wherein a mass ratio of the polyethylene glycol derivative I to the polyethylene glycol derivative II is 1: (0.8-1.2), preferably 1: (0.9-1.1), and more preferably 1: (0.95-1.05).

3. The medical hydrogel according to claim 1 or 2, wherein a mass ratio of the polyethylene glycol derivative I to trilysine is 1: (0.003-0.1), preferably 1: (0.005-0.05), and more preferably 1: (0.01-0.02).

4. A method of preparing the medical tissue adhesive according to any one of claims 1-3, wherein the method includes:

a step of preparing a solution A comprising a polyethylene glycol derivative I;
a step of preparing a solution B comprising a polyethylene glycol derivative II and trilysine; and
a step of mixing the solution A and the solution B, to obtain the medical tissue adhesive.

5. The method according to claim 4, wherein the step of preparing a solution B comprising a polyethylene glycol derivative II and trilysine includes:

preparing a solution comprising the polyethylene glycol derivative II and a solution comprising trilysine respectively, and mixing the two solutions, to obtain the solution B; or
preparing a solution comprising the polyethylene glycol derivative II, and adding trilysine to the solution comprising the polyethylene glycol derivative II, to obtain the solution B; or,
preparing a solution comprising trilysine, and adding the polyethylene glycol derivative II to the solution comprising trilysine, to obtain the solution B; or
mixing the polyethylene glycol derivative II and trilysine, and adding them to a solvent, to obtain the solution B.

6. The method according to claim 4 or 5, wherein the solution B comprises the polyethylene glycol derivative II in a percent concentration by mass of 10%-30% (m/v), and preferably 18%-22% (m/v).

7. The method according to claim 4 or 5, wherein the solution B comprises trilysine in a percent concentration by mass of 0.06%-2% (m/v), preferably 0.1%-1% (m/v), and more preferably 0.2%-0.4% (m/v).

8. The method according to claim 4 or 5, wherein the solution A comprises the polyethylene glycol derivative I in a percent concentration by mass of 10%-30% (m/v), and preferably 18%-22% (m/v).

9. Use of the medical tissue adhesive according to any one of claims 1-3 or a medical tissue adhesive obtained by the method according to any one of claims 4-8 as a biomedical material or in the preparation of a biomedical material; optionally, the biomedical material is at least one selected from (a)-(d):

(a) a medical adhesive for endoscope closure;
(b) a medical adhesive for blood vessel closure;
(c) a medical adhesive for surgery closure; and
(d) a medical adhesive for cerebrospinal fluid closure.

10. A composition for preparing a medical tissue adhesive, wherein the composition includes:

a component E comprising a polyethylene glycol derivative I with a structure as shown in Formula (I):

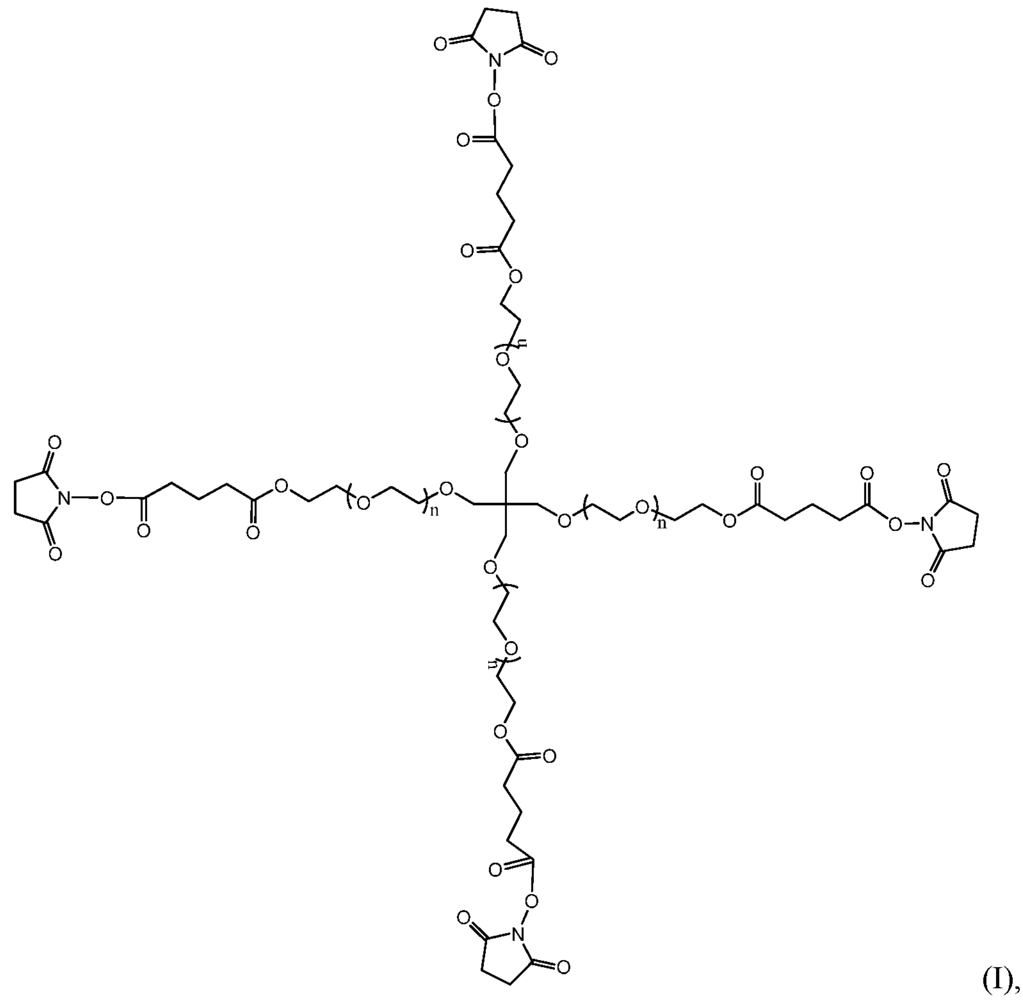

(I),

a component F comprising a polyethylene glycol derivative II with a structure as shown in Formula (II):

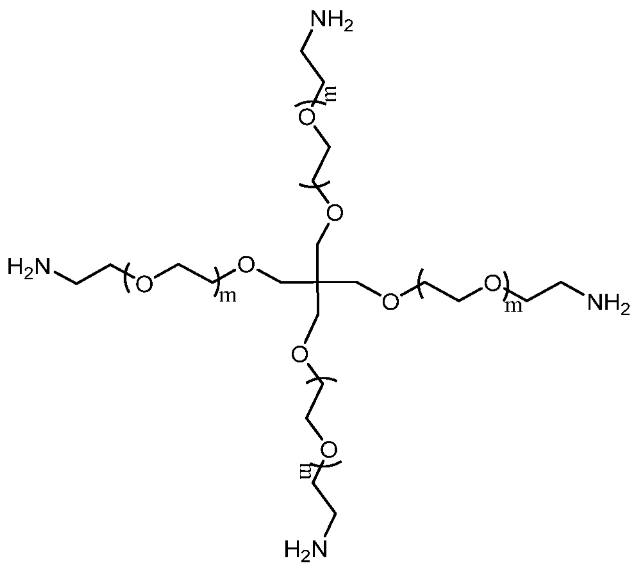

(II),

and
a component G comprising trilysine;
wherein m and n are each a natural number; a molecular weight of the polyethylene glycol derivative I is

2,000-20,000, preferably 5,000-15,000, and more preferably 8,000-12,000; a molecular weight of the polyethylene glycol derivative II is 2,000-20,000, preferably 5,000-15,000, and more preferably 8,000-12,000; optionally, any one of the component E, the component F, and the component G is a powder component or a liquid component.

**11.** A kit for tissue closure, wherein the kit includes the composition according to claim 10.

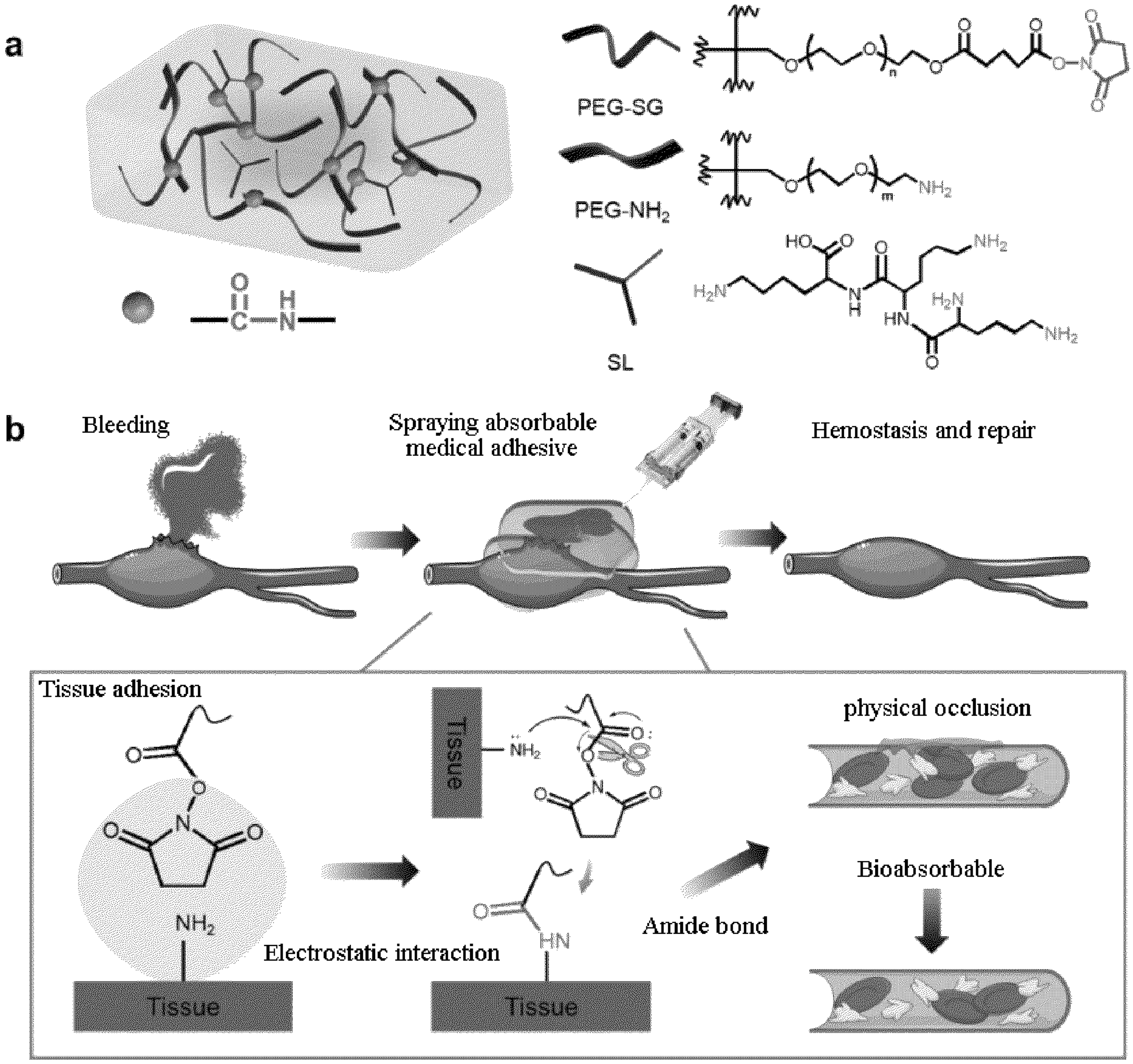
a
PEG-SG
PEG-NH2
SL
b
Bleeding
Spraying absorbable medical adhesive
Hemostasis and repair
Tissue adhesion
Tissue
Electrostatic interaction
Amide bond
physical occlusion
Bioabsorbable

FIG. 1

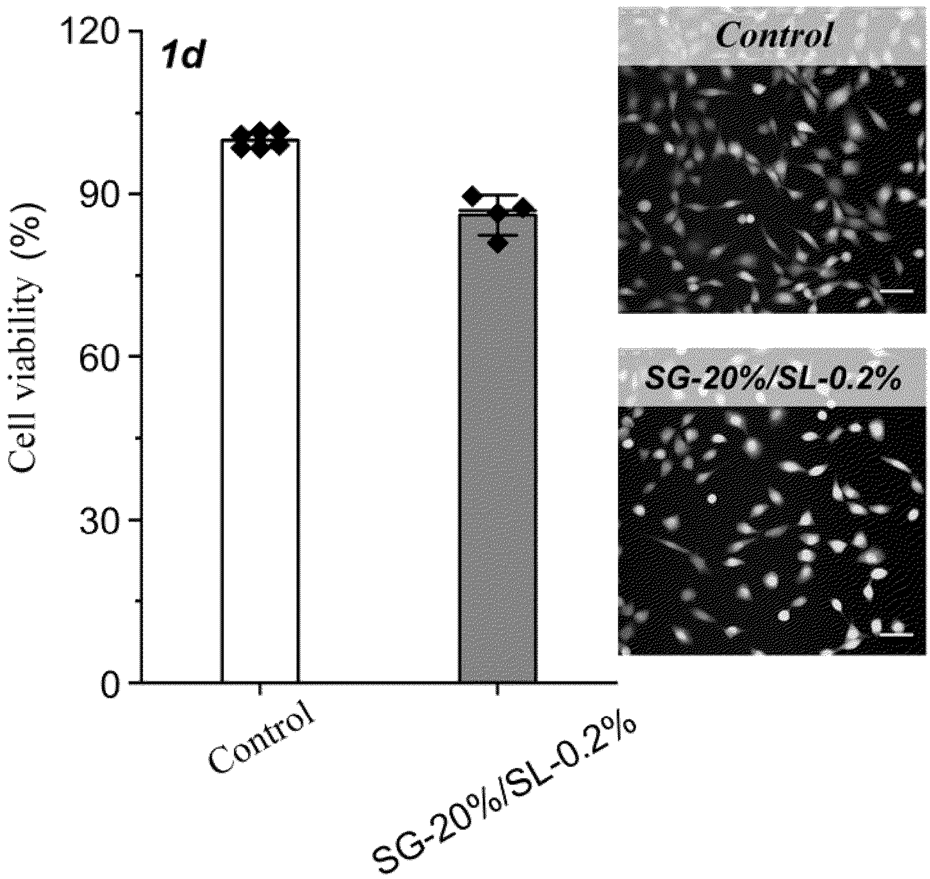
1d
Cell viability (%)
120
90
60
30
0
Control
SG-20%/SL-0.2%
Control
SG-20%/SL-0.2%

FIG. 2

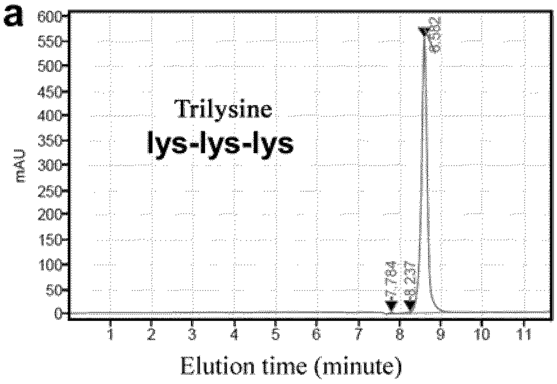


| Elution time (minute) | Area ratio (%) |
|---|---|
| 7.784 | 0.39 |
| 8.237 | 0.37 |
| 8.582 | 99.25 |

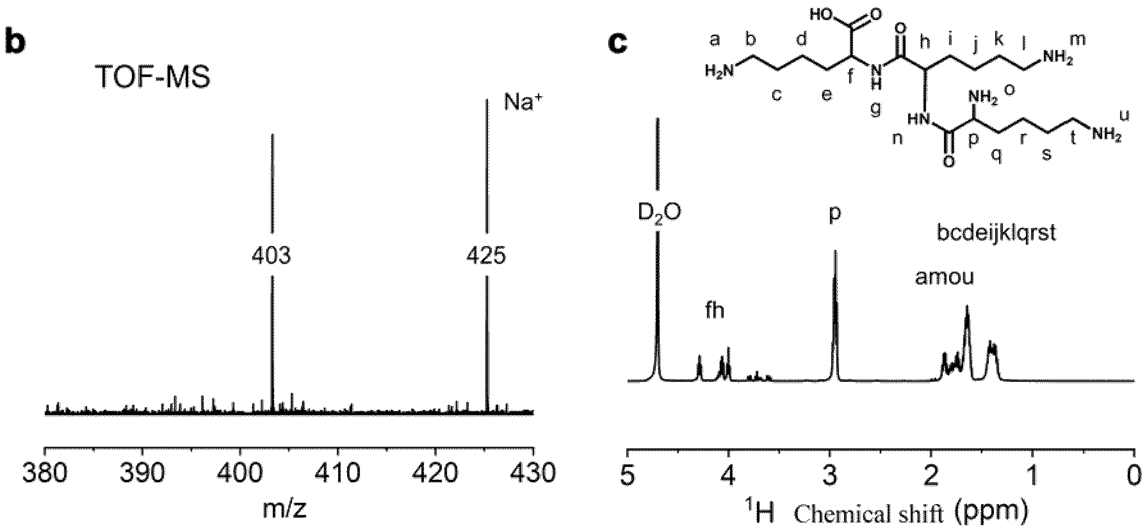


FIG. 3

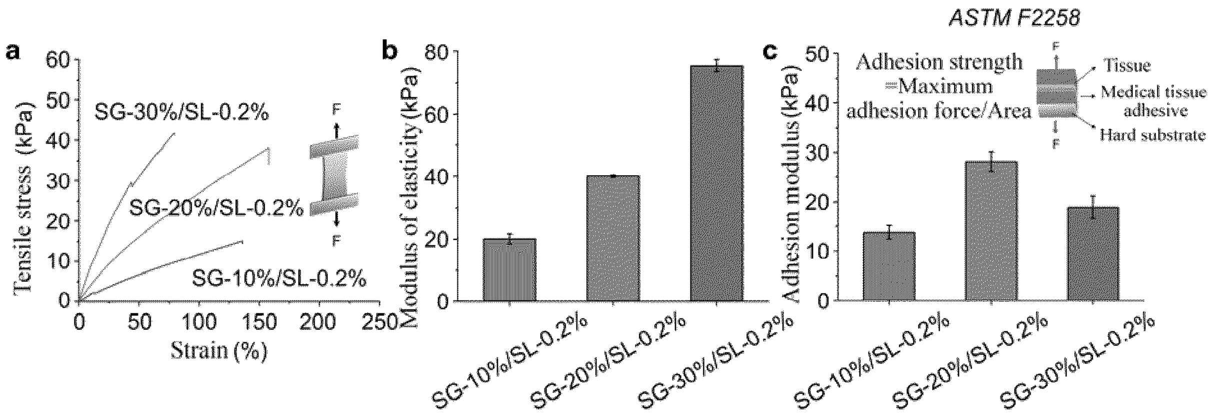


FIG. 4

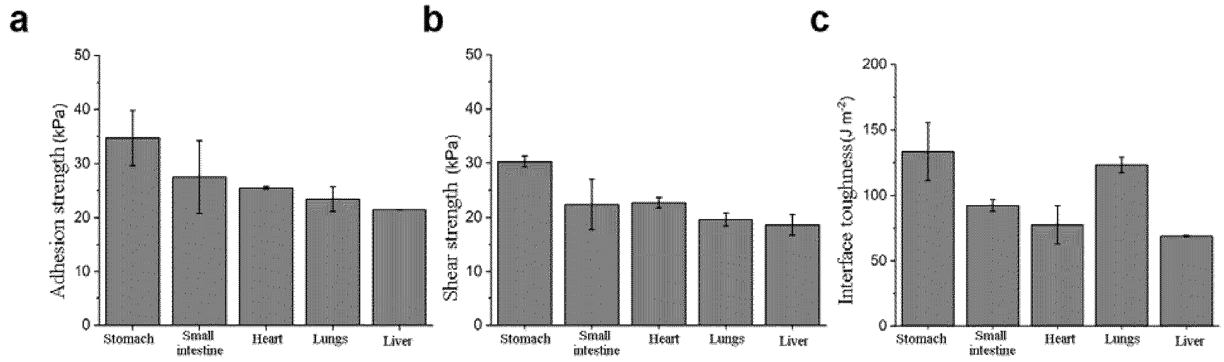


FIG. 5

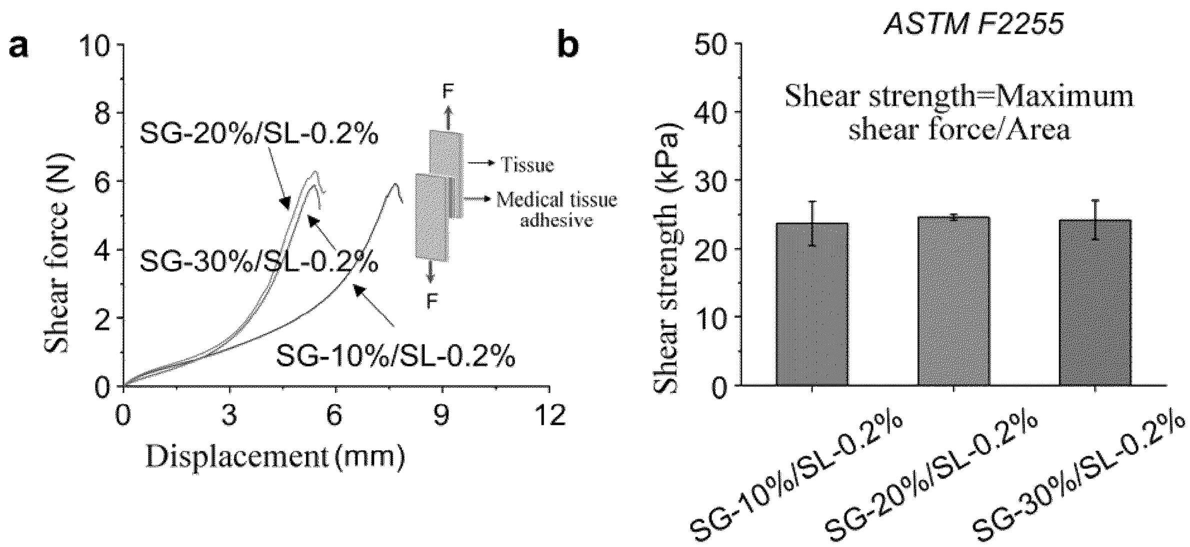


FIG. 6

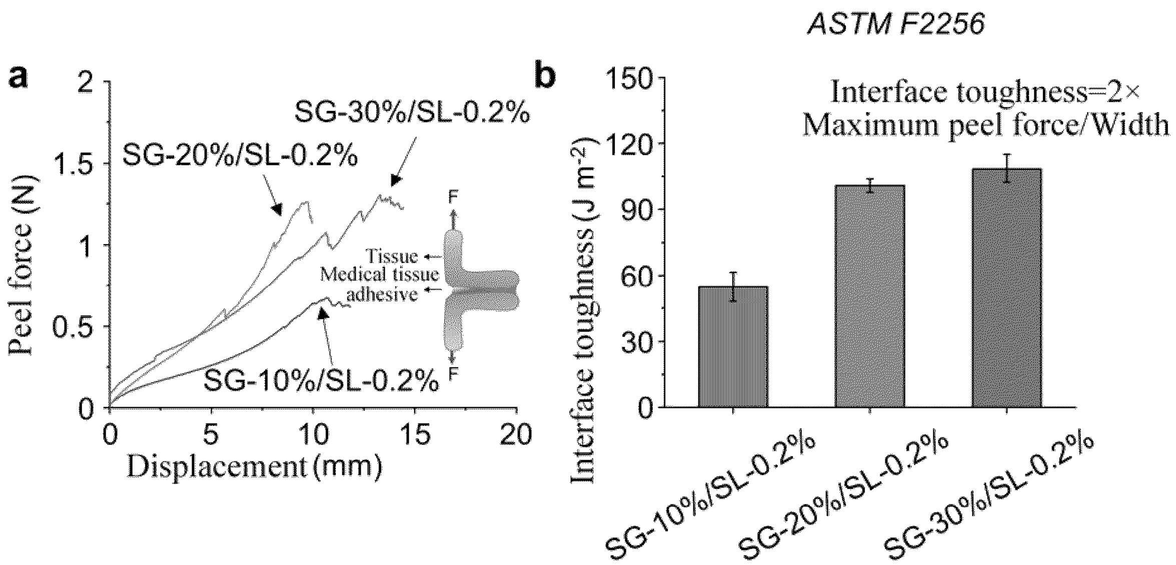


FIG. 7

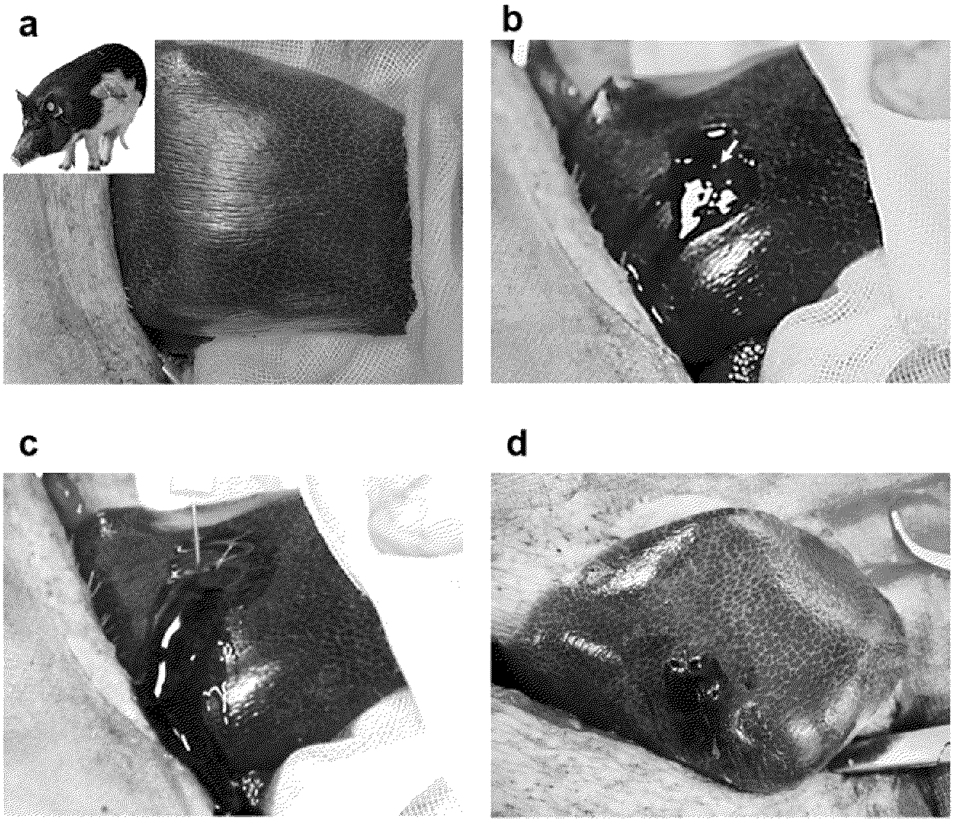


FIG. 8

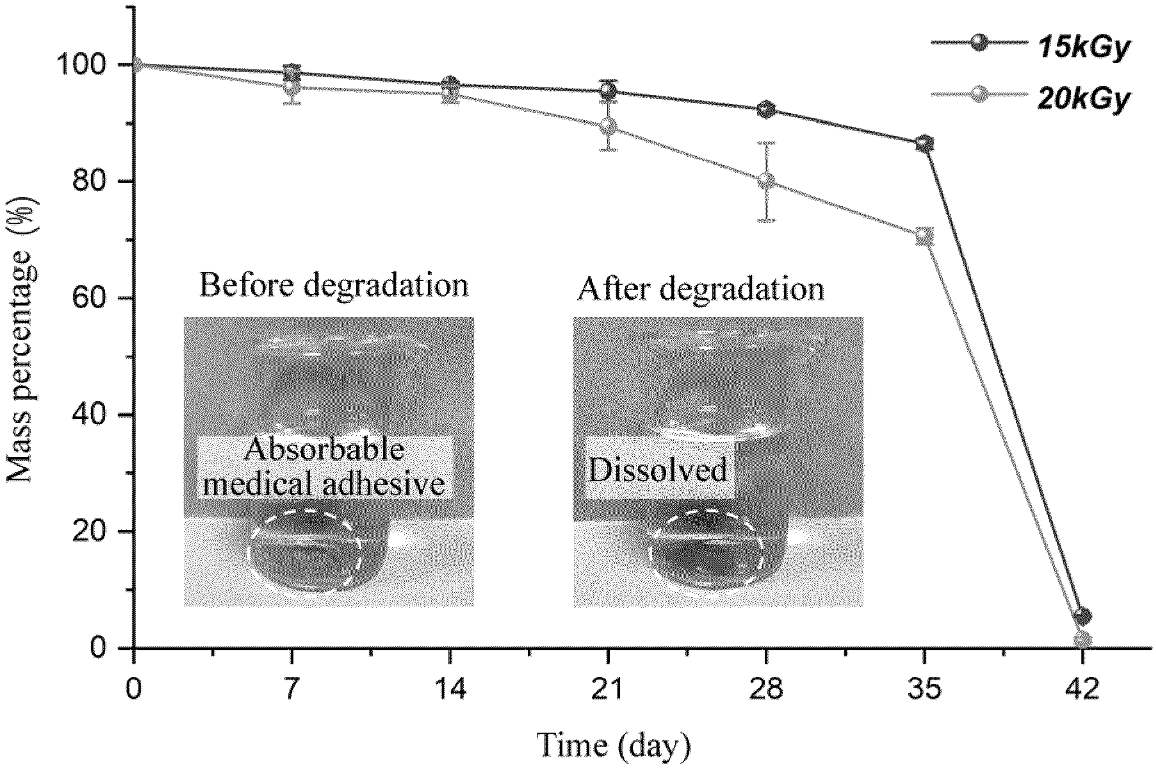
15kGy
20kGy
Before degradation
After degradation
Absorbable
medical adhesive
Dissolved
Mass percentage (%)
100
80
60
40
20
0
0
7
14
21
28
35
42
Time (day)

**FIG. 9**

## INTERNATIONAL SEARCH REPORT

International application No.
**PCT/CN2022/127941**

### A. CLASSIFICATION OF SUBJECT MATTER

A61L 24/04(2006.01)i； A61L 27/50(2006.01)i； A61L 27/26(2006.01)i； C08L 71/02(2006.01)i； A61L 24/00(2006.01)i； A61L 27/52(2006.01)i； C08J 3/075(2006.01)i

According to International Patent Classification (IPC) or to both national classification and IPC

### B. FIELDS SEARCHED

Minimum documentation searched (classification system followed by classification symbols)

A61L,C08L,C08J

Documentation searched other than minimum documentation to the extent that such documents are included in the fields searched

Electronic data base consulted during the international search (name of data base and, where practicable, search terms used)

CNKI, CNABS, SIPOABS, DWPI, CNTXT, ENTXT: 组织, 胶, 聚乙二醇, 四臂, 4臂, 琥珀酰亚胺戊二酸酯, 丁二酰亚胺戊二酸酯, PEG-SG, 胺, 氨基, PEG-NH2, 三赖氨酸, SL, 交联, 水凝胶, tissue, glue, polyethylene Glycol, four arms, 4 w arms, succinimide glutarate, amine, amino, trilysine, crosslink+, hydrogel

### C. DOCUMENTS CONSIDERED TO BE RELEVANT

| Category* | Citation of document, with indication, where appropriate, of the relevant passages | Relevant to claim No. |
|---|---|---|
| X | CN 102911493 A (SHANDONG SUCCESS PHARMACEUTICAL TECHNOLOGY CO., LTD.) 06 February 2013 (2013-02-06)<br>claims 1-10, and description, paragraph [0100] | 1-11 |
| X | CN 111420126 A (JIANGSU DIYUN MEDICAL TECHNOLOGY CO., LTD.) 17 July 2020 (2020-07-17)<br>claims 1-10 | 1-11 |
| X | CN 110643057 A (SAIKESAISI BIOTECHNOLOGY CO., LTD.) 03 January 2020 (2020-01-03)<br>claims 1-10, and description, embodiments 23 and 26 | 1-11 |
| X | WO 2010134988 A1 (HNOJEWYJ OLEXANDER) 25 November 2010 (2010-11-25)<br>claims 1-21 | 1-11 |
| A | CN 110433344 A (NKD PHARMA CO., LTD.) 12 November 2019 (2019-11-12)<br>entire document | 1-11 |

☐ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **08 December 2022** | **06 January 2023** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**
**Information on patent family members**

International application No.
**PCT/CN2022/127941**

| Patent document cited in search report | Publication date (day/month/year) | Patent family member(s) | Publication date (day/month/year) |
|---|---|---|---|
| CN 102911493 A | 06 February 2013 | None | |
| CN 111420126 A | 17 July 2020 | None | |
| CN 110643057 A | 03 January 2020 | None | |
| WO 2010134988 A1 | 25 November 2010 | None | |
| CN 110433344 A | 12 November 2019 | None | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202111520296